# EUROPEAN PATENT SPECIFICATION

(11) **EP 3 402 889 B1**
(45) Date of publication and mention of the grant of the patent: **27.10.2021**
(21) Application number: 17701908.0
(22) Date of filing: 11.01.2017
(51) Int. Cl.: C12N 15/86

(54) **COMBINING ADENOVIRUS AND CHEMOTHERAPEUTIC AGENTS FOR TREATING CANCER**
KOMBINATION VON ADENOVIRUS UND CHEMOTHERAPEUTIKA ZUR BEHANDLUNG VON KREBS
ASSOCIATION ADÉNOVIRUS/AGENTS CHIMIOTHÉRAPEUTIQUES POUR LE TRAITEMENT DU CANCER

(30) Priority: 15.01.2016 FI 20165026
(43) Date of publication of application: 21.11.2018
(60) Divisional application: 21194362.6
(73) Proprietor: Targovax Oy, 02600 Espoo (FI)
(72) Inventor: KURYK, Lukasz, 00180 Helsinki (FI); RANKI, Tuuli, 11130 Riihimäki (FI); PESONEN, Sari, 00980 Helsinki (FI); HAAVISTO, Elina, 00570 Helsinki (FI); VUOLANTO, Antti, 02880 Veikkola (FI); VASSILEV, Lotta, 00270 Helsinki (FI)
(74) Representative: Boco IP Oy Ab
(86) International application number: PCT/FI2017/050010
(87) International publication number: WO 2017/121925

(56) References cited:
- WO-A1-2010/072900
- LIIKANEN I ET AL: "Oncolytic Adenovirus With Temozolomide Induces Autophagy and Antitumor Immune Responses in Cancer Patients", MOLECULAR THERAPY, vol. 21, no. 6, 1 June 2013 (2013-06-01), pages 1212-1223, XP055362204, US ISSN: 1525-0016, DOI: 10.1038/mt.2013.51 -& Liikanen I ET AL: "Supplementary Text and Figures", , 1 June 2013 (2013-06-01), XP055362210, Retrieved from the Internet: URL:http://www.sciencedirect.com/science/M iamiMultiMediaURL/1-s2.0-S1525001616326156 /1-s2.0-S1525001616326156-mmc1.pdf/270195/ html/S1525001616326156/73ecc46c9018fa7aaaf 2fca949ed101c/mmc1.pdf [retrieved on 2017-04-05]
- KURYK L ET AL: "Synergistic anti-tumor efficacy of immunogenic adenovirus ONCOS-102 (Ad5/3-D24-GM-CSF) and standard of care chemotherapy in preclinical mesothelioma model", INTERNATIONAL JOURNAL OF CANCER, vol. 139, no. 8, 15 October 2016 (2016-10-15), pages 1883-1893, XP055362219, US ISSN: 0020-7136, DOI: 10.1002/ijc.30228
- RANKI T ET AL: "Local treatment of a pleural mesothelioma tumor with ONCOS-102 induces a systemic antitumor CD8 + T-cell response, prominent infiltration of CD8 + lymphocytes and Th1 type polarization", ONCOIMMUNOLOGY, vol. 3, no. 10, 1 September 2014 (2014-09-01), page e958937, XP055362225, DOI: 10.4161/21624011.2014.958937
- BOISGERAULT N ET AL: "Oncolytic virotherapy for human malignant mesothelioma: recent advances", ONCOLYTIC VIROTHERAPY, 1 September 2015 (2015-09-01), page 133, XP055362222, DOI: 10.2147/OV.S66091
- SIURALA M ET AL: "Oncolytic adenovirus and doxorubicin-based chemotherapy results in synergistic antitumor activity against soft-tissue sarcoma", INTERNATIONAL JOURNAL OF CANCER, vol. 136, no. 4, 15 February 2015 (2015-02-15), pages 945-954, XP055362230, US ISSN: 0020-7136, DOI: 10.1002/ijc.29048

### SEQUENCE LISTING

<110> Targovax Oy
<120> COMBINATING ADENOVIRUS AND CHEMOTHERAPEUTIC AGENTS FOR TREATING CANCER
<130> B5587PC
<160> 9
<170> BiSSAP 1.3
<210> 1
   <211> 18
   <212> DNA
   <213> Artificial Sequence
<220>
   <223> Oligonucleotide
<400> 1
   ggagtgcgcc gagacaac 18
<210> 2
   <211> 19
   <212> DNA
   <213> Artificial Sequence
<220>
   <223> Oligonucleotide
<400> 2
   actacgtccg gcgttccat 19
<210> 3
   <211> 28
   <212> DNA
   <213> Artificial Sequence
<220>
   <223> Oligonucleotide
<400> 3
   tggcatgaca ctacgaccaa cacgatct 28
<210> 4
   <211> 16
   <212> DNA
   <213> Artificial Sequence
<220>
   <223> Oligonucleotide
<400> 4
   cgagcggttc cgatgc 16
<210> 5
   <211> 20
   <212> DNA
   <213> Artificial Sequence
<220>
   <223> Oligonucleotide
<400> 5
   tggatgccac aggattccat 20
<210> 6
   <211> 27
   <212> DNA
   <213> Artificial Sequence
<220>
   <223> Oligonucleotide
<400> 6
   aggctctttt ccagccttcc ttcttgg 27
<210> 7
   <211> 21
   <212> DNA
   <213> Artificial Sequence
<220>
   <223> Oligonucleotide
<400> 7
   cagcagatgt ggatcagcaa g 21
<210> 8
   <211> 21
   <212> DNA
   <213> Artificial Sequence
<220>
   <223> Oligonucleotide
<400> 8
   ctagaagcat ttgcggtgga c 21
<210> 9
   <211> 22
   <212> DNA
   <213> Artificial Sequence
<220>
   <223> Oligonucleotide
<400> 9
   aggagtatga cgccggcccc tc 22

## Description

### FIELD OF THE INVENTION

The present invention relates to the field of medicine. Specifically, the present invention relates to a novel strategy of using ONCOS-102 adenovirus for the treatment of human cancer in combination with two chemotherapeutic agents, either Pemetrexed and Cisplatin, or Pemetrexed and Carboplatin. Also methods of using this combination of virus and chemotherapeutic agents in the treatment of malignant mesothelioma are disclosed. Other medications in addition to the virus and two chemotherapeutic agents can also be included into the treatment protocol.

### BACKGROUND OF THE INVENTION

Malignant mesothelioma (MM) is an aggressive and a rare form of cancer that develops from mesothelium. MM is primary caused by exposure to asbestos and exhibits a long latency period, usually >30 years. The median survival time for mesothelioma patients after diagnosis is typically only 9-12 months. MM affects the pleura (the outer lining of the lungs and internal chest wall) in 85.5% of MM cases, peritoneum (the lining of the abdominal cavity) in 13.2% of MM cases, pericardium (the sac that surrounds the heart) in 0.5% of MM cases, and tunica vaginalis (a sac that surrounds the testis) in 0.8% of MM cases. MM tumors are often poorly responsive to standard therapies and incidence is constantly increasing worldwide. The low incidence of MM has for a long time limited the discovery of new drugs, thus new treatment modalities are highly needed.

Typically, cancer is treated with conventional treatment regimens such as surgery, hormonal therapies, chemotherapies, radiotherapies and/or other therapies. However, in many cases cancers which often are characterized by an advanced stage cannot be cured with present therapies.

Virotherapy is a relatively novel treatment approach, which harnesses the natural ability of some viruses to kill the cells in which they proliferate and the ability to spread to neighboring cells, thereby amplifying the therapeutic effect of the initial input dose. In virotherapy the cancer cell transduction and viral replication are carefully controlled by genetic engineering of the viral genome to gain effective and safe tumor eradication. Safe tumor eradication necessitates introduction of various genetic modifications to the adenoviral genome, thereby restraining replication exclusively to tumor cells and eventually obtaining selective eradication of the tumor without side effects to healthy tissue.

Specific deletions on adenoviral key regulatory genes can be utilized to create dysfunctional proteins or the lack of their expression that leads to dependence on a specific genetic feature present in target cells. Partial deletions of *E1A* result in restricted replication in normal cells but allow replication in target cells, such as cancer cells. Conditionally replicating viruses featuring a 24 base pair deletion in the CR2 (constant region 2) were created and shown to be potent and selective in the treatment of glioma and breast cancer xenografts (Fueyo et al. 2000; Heise et al. 2000). Their cancer specificity results from the inability of dysfunctional E1A to release E2F1 transcription factor, which leads to the requirement of free E2F1. E2F1 is abundant in cancer cells, where the pRb pathway is most often disrupted (Hanahan and Weinberg 2000).

Clinical and preclinical results have shown that treatment with unarmed oncolytic viruses is not immunostimulatory enough to result in sustained anti-tumoral therapeutic immune responses. In this regard, oncolytic viruses have been armed to be more immunostimulatory. Viruses can be engineered to express highly immunogenic proteins such as granulocyte-macrophage colony-stimulating factor (GM-CSF). When immunogenic proteins are expressed within tumor microenvironment, they are potent stimulators of specific and long-lasting antitumor immunity. Introduction of immunotherapeutic genes into tumor cells and, furthermore, their translation into proteins, leads to the activation of the immune response and to more efficient destruction of tumor cells. The most relevant immune cells in this regard are natural killer cells (NK) and cytotoxic CD8+ T-cells.

ONCOS-102 (Ad5/3-D24-GM-CSF; disclosed in WO 2010/072900) is a serotype 5 adenovirus, comprising a chimeric capsid for enhanced gene delivery to cancer cells and a 24 bp deletion in Rb binding site of E1A region for cancer cell restricted replication. ONCOS-102 is armed with granulocyte-macrophage colony-stimulating factor (GM-CSF) for an enhanced immuno-stimulatory effect. Safety and immunological activity of ONCOS-102 has already been demonstrated in phase 1 clinical study (NCT01598129). In this phase 1 study, local treatment of pleural mesothelioma with ONCOS-102 induced a systemic anti-tumor CD8+ T cell response and infiltration of CD8+ T cells into tumors in the last line refractory malignant pleural mesothelioma patient.

Koski et al. (2010) discloses treating total of 21 patients with advanced solid tumors refractory to standard therapies with ONCOS-102. According to those studies ONCOS-102 seems safe in treating cancer patients. Also promising signs of efficacy were seen.

Publication WO 99/59604 discloses a method for treating squamous cell cancer consisting of direct injection of adenovirus into the cancer and administration of two chemotherapeutics, cisplatin and 5-fluorouracil.

Siurala et al. (2015) discloses a combination of a capsid-modified oncolytic adenovirus CGTG-102 (same as ONCOS-102) with doxorubicin, with or without ifosfamide. When tested *in vivo* against established soft-tissue sarcoma tumors in fully immunocompetent Syrian hamsters, the combination was found to be effective.

Publication US 7393478 B2 discloses a method for inhibiting tumor growth in a subject comprising administering to the subject an effective amount of a micelle comprising cisplatin and a therapeutic agent. Therapeutic agent in this method can be for example GM-CSF.

In practice, the treatment of malignant mesothelioma (MM) has remained unchanged since 2003. The most commonly used first-line chemotherapy for MM is performed with chemotherapeutic agents Pemetrexed (Pemetrexed Disosium, Alimta), Cisplatin (Platinol), Carboplatin (Paraplatin) and their combinations. Despite progress in conventional cancer treatment regimens, human mesothelioma remains incurable and new treatment alternatives are desperately needed.

### SUMMARY OF THE INVENTION

Objects of the present disclosure are to provide a novel combination of oncolytic adenovirus and chemotherapeutic agents, a novel combination therapy using oncolytic adenovirus and chemotherapeutic agents for treating cancer in a patient and also to solve problems relating to conventional cancer therapy.

An aspect of the invention is ONCOS-102 adenovirus for use in the treatment of human malignant mesothelioma, wherein administering the virus to a human patient in need thereof is done in combination with administering two chemotherapeutic agents, either Pemetrexed and Cisplatin, or Pemetrexed and Carboplatin.

### BRIEF DESCRIPTION OF THE DRAWINGS

The following figures are included to further demonstrate certain aspects and features of the present invention. The invention may be better understood by reference to one or more of these drawings in combination with the detailed description of specific embodiments, including examples.

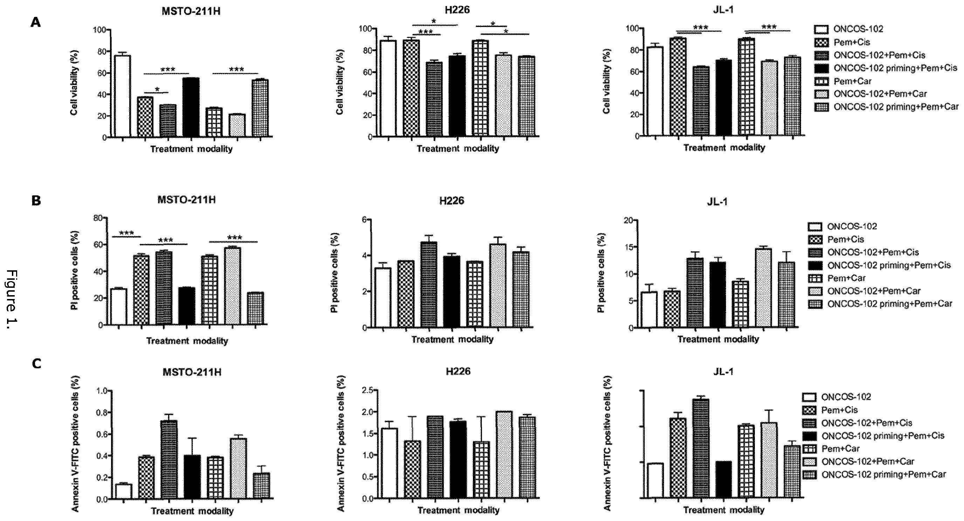

**Figure 1****.** Treatment of human mesothelioma cells: JL-1, MSTO-211H and H226 with Pemetrexed and Cisplatin or Pemetrexed and Carboplatin alone or in combination with ONCOS-102 (10VP/cell), or with ONCOS-102 alone in *in vitro* efficacy study. a) Antitumor efficacy was measured by MTS cell-viability assay. Cell viability was determined against untreated cells (mock) 72 hours after treatment. The amount of b) necrotic cells (PI) and c) early apoptotic cells (FITC-labeled Annexin-V) 48 hour after treatment were analyzed by flow cytometry. Error bars, mean±SEM: ^{∗}p < 0.05, ^{∗∗}p < 0.01, ^{∗∗∗}p < 0.001.

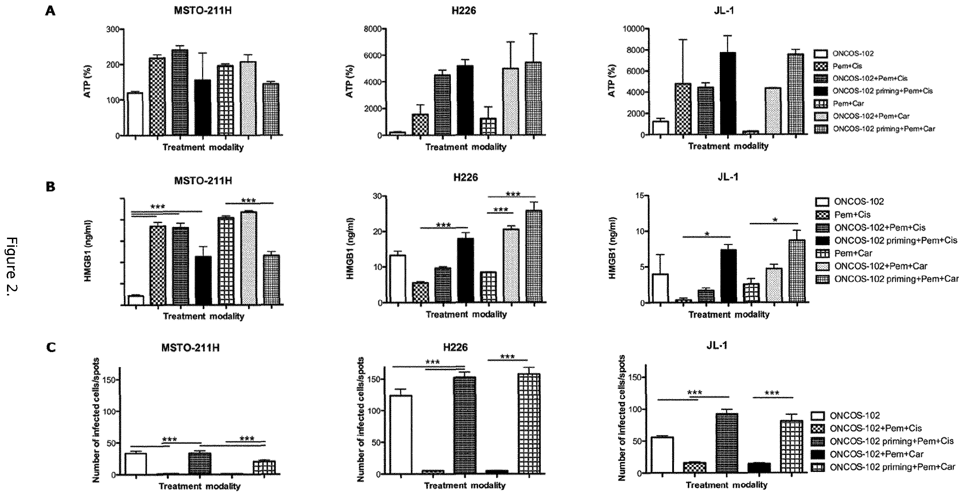

**Figure 2****.** Combined treatment of human mesothelioma cells: JL-1, MSTO-211H and H226 with Pemetrexed and Cisplatin or Pemetrexed and Carboplatin alone or in combination with ONCOS-102 (10VP/cell), or with ONCOS-102 alone - *in vitro* immunogenic tumor cell death. a) Extracellular ATP was measured from supernatant 48 (Jl-1, MSTO-211H) and 72 (H226) hours after treatment using ATP determination kit. b) Extracellular HMGB1 secretion into the supernatant was measured 3 days after treatment utilizing ELISA assay. c) Calreticulin exposure on outer cell surface of tested human mesothelioma cells was measured 24 (H226, JI-1) and 48 (MSTO-211H) hours after treatment by flow cytometry. Error bars, mean±SEM: ^{∗}p < 0.05, ^{∗∗}p < 0.01, ^{∗∗∗}p < 0.001.

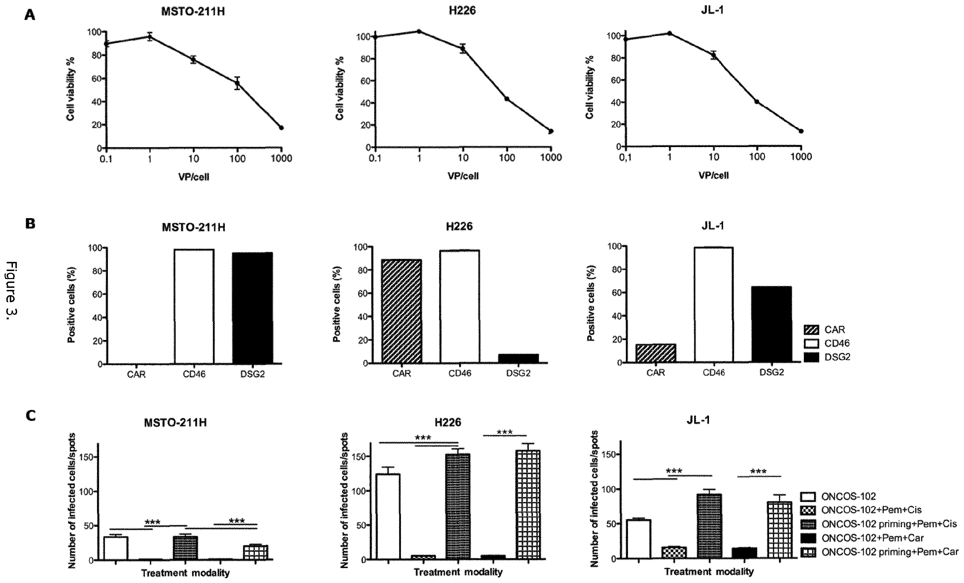

**Figure 3****.** Oncolytic efficacy of ONCOS-102, receptor expression profile and virus infectivity assay measured in three mesothelioma cell lines (Jl-1, MSTO-211H, H226). a) Oncolytic efficacy of ONCOS-102 (0,1; 1; 10; 100 and 1000 VP/cell) was measured by MTS cell-viability assay 3 days after treatment initialization. Cell viability profile was determined against untreated cells (mock). b) The expression of CAR, DSG2 and CD46 on the mesothelioma cell lines was measured by flow cytometry after specific antibody staining. c) ONCOS-102 infectivity assay was performed for five treatment groups. The determination of the ONCOS-102 infectivity was based on visual quantification of infected cells after the staining of virus hexon protein and finally the calculation for detected spots. For each 5 replicates 5 images of non-overlapping fields have been acquired using an AMG EVO XL microscope. For infectivity comparison, the data have been presented as the average number of spots in 5 wells. Error bars, mean±SEM: ^{∗}p < 0.05, ^{∗∗}p < 0.01, ^{∗∗∗}p < 0.001.

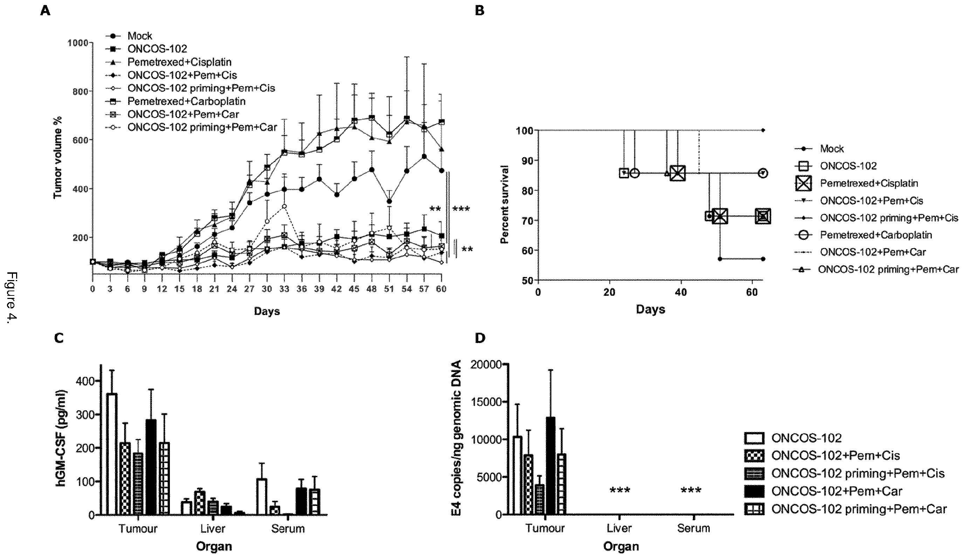

**Figure 4****.** Antitumor efficacy of ONCOS-102 (1E+8 VP/mouse intratumorally (i.t.)) in combination with Pemetrexed (10 mg/kg in 100 µl intraperitoneal (IP)), Cisplatin (1.5 mg/kg in 100 µl IP) or Carboplatin (8 mg/kg in 100 µl IP) in human mesothelioma xenograft model in BALB/c nude mice (7 mice per group, 14 tumors per group) was analyzed and compared to mock or each treatment alone. a) BABL/c nude mice bearing s.c. H226 tumors (6×10⁶ cells/tumor) were treated according to the treatment scheme (Table 1) with ONCOS-102 (i.t.) and chemotherapies (IP) in 8 groups. Animals were treated every 3 days throughout 2 months. b) Survival profile was calculated by Kaplan-Meier test. c) Human GM-CSF produced by ONCOS-102 was analyzed from tumor, liver and serum after animal euthanasia utilizing ELISA technique. d) Adenoviral copies (E4 gene) were measured by qPCR from tumor, liver and serum of euthanized animals. Error bars, mean±SEM: ^{∗}p < 0.05, ^{∗∗}p < 0.01, ^{∗∗∗}p < 0.001.

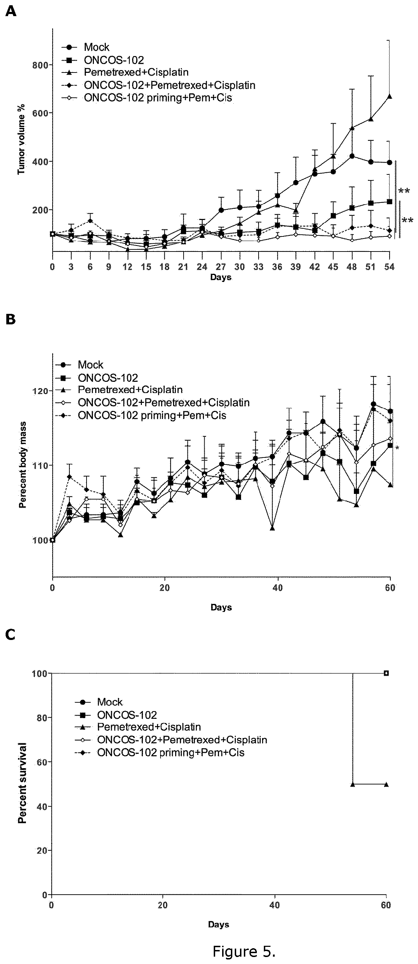

**Figure 5****.** Antitumor efficacy of ONCOS-102 (1E+8 VP/mouse i.t.), Pemetrexed (10 mg/kg in 100 µl IP) and Cisplatin (1.5 mg/kg in 100 µl IP), combination of all three or combination of the chemotherapeutics in human mesothelioma xenograft model in BALB/c nude mice (2 mice per group, 2 tumors per mouse). a) BABL/c nude mice bearing s.c. H226 tumors (7×10⁶ cells/tumor) were treated with ONCOS-102 (i.t.) and chemotherapy (IP) in 4 groups. Animals were treated every 3 days throughout 54 days. b) Body weigh was monitored and measured throughout the experiment. Weight (g) was converted to the parentage value of body weight, where body mass at day zero was set as 100%. c) Survival profile was calculated by Kaplan-Meier test. Error bars, mean±SEM: ^{∗}p < 0.05, ^{∗∗}p < 0.01, ^{∗∗∗}p < 0.001.

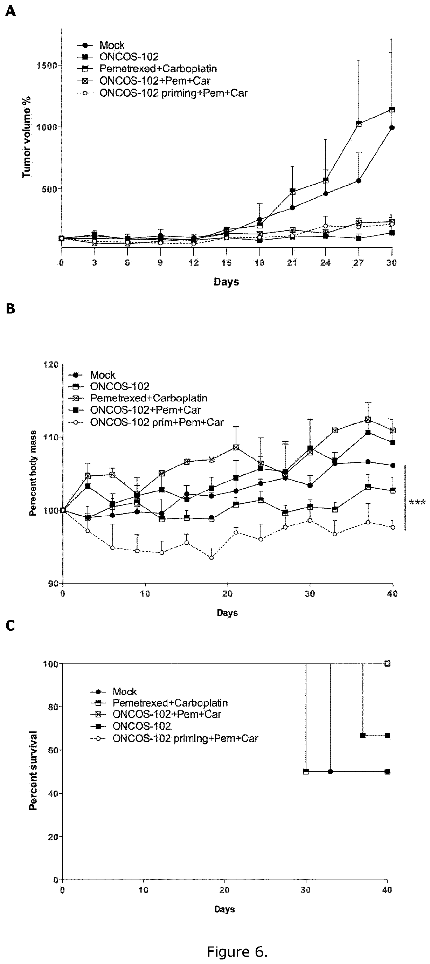

**Figure 6****.** Antitumor efficacy of ONCOS-102 (2E+8 VP/mouse i.t.), Pemetrexed (6.7 mg/kg in 100 µl IP) and Carboplatin (5.4 mg/kg in 100 µl IP), combination of all three or combination of the chemotherapeutics in human mesothelioma xenograft model in BALB/c nude mice (2 mice per group, 2 tumors per mouse). a) BALB/c nude mice bearing s.c. H226 tumors (7×10⁶ cells/tumor) were treated with ONCOS-102 (i.t.) and chemotherapy (IP) in 4 groups. Animals were treated every 3 days throughout 30 days. b) Body weight was monitored and measured throughout the experiment. Weight (g) was converted to the parentage value of body weight, where body mass at day zero was set as 100%. c) Survival profile was calculated by Kaplan-Meier test. Error bars, mean±SEM: ^{∗}p < 0.05, ^{∗∗}p < 0.01, ^{∗∗∗}p < 0.001.

### Detailed description of the invention

Unless defined otherwise, all technical and scientific terms used in this application have the same meaning as commonly understood by one of ordinary skill in the art to which this invention pertains.

Mesothelioma is a treatment-resistant cancer to standard therapies with constantly increasing amount of mortal cases worldwide. The lungs in the chest, as well as abdominal organs such as the stomach, the intestines, the liver, and the heart are each wrapped in membranes such as pleura, peritoneum, and pericardium. A portion that covers the surface of such a membrane is referred to as mesothelium. A tumor that is developed from such mesothelium is referred to as a mesothelioma. There are three main histological subtypes of malignant mesothelioma: epithelioid, sarcomatous, and biphasic. "Malignant mesothelioma" (MM) as used herein refers to mesothelioma affecting pleura, peritoneum, pericardium or tunica vaginalis The most effective standard treatment against MM to date is a combination of Pemetrexed and Cisplatin, which leads to 41% response rate (RR). The combination of Cisplatin and Pemetrexed improves the survival of mesothelioma patients compared to single-agent chemotherapy. However, MM is still a lethal disease with a prognosis of median PFS/OS from the initiation of treatment up to 12 months, and therefore new treatment modalities are needed.

The term "anti-viral response" as used herein refers to a cell's response to viral infection and includes, for example, production of interferons, cytokine release, production of chemokines, production of lymphokines or any combination thereof.

The expressions "normal host cell" and "normal tissue" as used herein refer to a non-cancerous, non-infected cell or tissue with an intact anti-viral response.

The term "oncolytic agent" as used herein refers to an agent capable of inhibiting the growth of and/or killing tumor cells.

The term "subject" as used herein refers to any living organism, including humans and animals, human and animal tissue, and human and animal cells.

The term "patient" as used herein refers to any subject (preferably human) afflicted with a disease, such as MM, who is likely to benefit from a treatment with combination therapy as described herein.

Adenoviruses are non-enveloped viruses 70-90 nm in diameter with an icosahedral capsid. Their genome is linear, double stranded DNA varying between 25-45 kilobases in size with inverted terminal repeats (ITRs) at both termini and a terminal protein attached to the 5'ends.

The icosahedral capsid is formed by three major proteins, of which the hexon trimers are most abundant. Each of the twelve vertices of the capsid also contains a pentameric protein, a penton base that is covalently attached to the fiber. The fiber is a trimeric protein that protrudes from the penton base and is a knobbed rod-like structure. Other viral proteins IIIa, IVa2, VI, VIII and IX are also associated with the viral capsid. The proteins VII, small peptide *mu* and a terminal protein (TP) are associated with DNA. Protein V provides a structural link to the capsid via protein VI.

As used herein the term "capsid" refers to the protein shell of the virus, which includes hexon, fiber and penton base proteins.

All human adenoviruses have similarities in their fiber architecture. Each has an N-terminal tail, a shaft with repeating sequences, and a C-terminal knob domain with a globular structure. The knob domain is principally responsible for binding the target cellular receptor and its globular structure presents a large surface for lateral and apical binding. The fiber proteins of adenoviruses from different subgroups most distinctively differ in length and ability to bend.

The fiber participates in attachment of the virus to the target cell. First, the knob domain of the fiber protein binds to the receptor of the target cell, secondly, the virus interacts with an integrin molecule, and thirdly, the virus is endocytosed into the target cell. Next, the viral genome is transported from endosomes into the nucleus and the replication of the viral genome can begin.

As used herein, "Ad5/3 chimerism" of the capsid refers to a chimerism, wherein the knob part of the fiber is from Ad serotype 3, and the rest of the fiber is from Ad serotype 5.

Adenoviruses are dependent on the cellular machinery to replicate the viral genome. They can infect quiescent cells and induce them into a cell cycle S-phase-like state enabling viral DNA replication. The adenoviral genome can be divided into immediate early (*E1A*), early *(E18, E2, E3, E4),* intermediate (*IX*, *Iva*), and late (*L1-L5*) genes.

E3 gene products are not essential for virus replication *in vitro,* but are dedicated to the control of various host immune responses. E3-gp19K inhibits the transport of the class 1 major histocompatibility complex (MHC) from the endoplasmic reticulum (ER) to the plasma membrane, thereby preventing the presentation of peptides to T lymphocytes by MHC.

The adenoviral E1A protein was originally described as a pRb binding protein capable of inducing DNA replication in quiescent normal cells. One of the key functions of E1A protein is to disrupt the pRb-E2F interactions, thereby releasing E2F transcription factors to activate the E2F responsive promoters and transcription of the genes they control, such as adenoviral E2A. The conserved region 2 (CR2) in E1A protein forms a strong interaction with the pocket binding domain of pRb and CR1 mediates the actual disruption of the E2F binding of pRb. Conditionally replicating viruses featuring a 24 base pair deletion in the CR2 were created and shown to be potent and selective in the treatment of glioma and breast cancer xenografts. Their cancer specificity results from the inability of dysfunctional E1A to release E2F1 transcription factor, which leads to the requirement of free E2F1.

ONCOS-102 adenovirus has been earlier disclosed in publication WO 2010/072900. ONCOS-102 is a serotype 5 adenovirus (Ad5) displaying the following modifications differing from the Ad5 genome:
1. A 24 base pair (bp) deletion in the E1A-gene constant region 2 (CR2). The dysfunctional E1A protein is unable to bind and release E2F1 transcription factor from the retinoblastoma protein (Rb), leading to the requirement of free E2F1 for adenovirus gene transcription. Free E2F1 is abundant in cancer cells, where the pRb pathway is most often disrupted. Thereby viruses with the 24 bp deletion in *E1A* are able to efficiently replicate in cancer cells. The *E1A* gene transcription into mRNA is being controlled by the endogenous *E1A* promoter.
2. A 965 bp deletion has been introduced in the early 3 (E3) region coding for 6.7K and gp19K proteins. These proteins are associated with the ability of adenovirus to evade host immune control mechanisms and their functions are expendable for adenoviral replication.
3. A transgene coding for the human granulocyte macrophage colony stimulating factor (GM-CSF) protein has been inserted to the E3 region, replacing 6.7K and gp19K. The GM-CSF gene transcription into mRNA is being controlled by the endogenous E3 promoter. In other words, in ONCOS-102 adenovirus 965 base pairs coding for the viral genes gp19K and 6.7K have been deleted from the E3 region and a transgene GM-CSF has been introduced to replace them.
4. The serotype 5 fiber knob has been replaced by serotype 3 fiber, thereby allowing entry of the virus to cells via the serotype 3 receptor instead of the serotype 5 receptor CAR.

In ONCOS-102 adenovirus the native E1A promoter is present, i.e. it has not been replaced by another promoter.

In short, in ONCOS-102 adenovirus, GM-CSF is under endogenous viral E3 control elements, which results in replication-associated transgene expression starting about 8 hours after infection. The virus replicates in a tumor-selective manner thus resulting in tumor-restricted production of GM-CSF. Tumor specificity is achieved by a 24-bp deletion, which abrogates the Rb-binding site of E1A and, as demonstrated in previous reports, the virus replicates selectively in cells with p16-Rb pathway defects, including most if not all human cancers. The oncolytic potency of ONCOS-102 adenovirus was shown to be more effective than the wild-type control virus.

Oncolytic adenoviruses that express GM-CSF induce anti-cancer immunity while acting directly on cancer cells by oncolysis. GM-CSF is a potent inducer of systemic anti-tumor immunity associated with recruitment and maturation of antigen presenting cells (APCs), mainly dendritic cells, as well as recruitment of cells of the innate immunity arm. However, systemically elevated cytokine levels represent a risk for toxic side-effects. Besides the direct risk of side effects mediated by high serums concentrations of GM-CSF, an indirect risk results from recruitment of myeloid derived suppressor cells (MDSC). While the immunosuppressive effect of MDSC is potentially harmful for cancer patients in general, it could be particularly counterproductive in the context of cancer immunotherapy. Thus restricting the GM-CSF expression to the tumor site is crucial.

ONCOS-102 adenovirus has shown a good oncolytic potential and production of functionally active human GM-CSF *in vitro* (Koski *et al.* 2010). It was shown in immune-competent hamsters that the virus is effective in deterring the growth of aggressive syngeneic pancreatic tumors. Evidence of replication of the virus in tumors was shown by measuring viral copy number. Selectivity of replication was demonstrated as there was no increase in viral copy numbers in directly injected liver tissue. Local replication-linked production of GM-CSF in tumors was demonstrated, while there was very little leakage of GM-CSF into serum or liver. It was also shown that combining low-dose cyclophosphamide with ONCOS-102 adenovirus can enhance antitumor effect, whereas cyclophosphamide treatment alone did not result in significant reduction of tumor growth.

Overall, treatment of advanced cancer patients with ONCOS-102 adenovirus appears to be safe and promising signs of possible efficacy have been observed. Although virus is present in serum for extended periods even after a single dose, multiple injections are likely to improve tumor transduction and enhance antitumor immunity.

"Chemotherapy" as used herein refers to the use of chemical compounds or drugs in the treatment of disease, though the term chemotherapy is most often associated with the treatment of cancer. Cancer chemotherapeutic compounds encompass nearly 100 individual drugs.

The most common side effects of chemotherapeutic agents are nausea and vomiting. A large proportion of individuals also suffer from myelosuppression, or suppression of the bone marrow, which produces red blood cells, white blood cells and platelets. These and other side effects are also exacerbated by the suppression of the immune system concomitant with the destruction and lack of production of white blood cells, and associated risk of opportunistic infection.

Other side effects common to a wide range of chemotherapeutic agents include: hair loss (alopecia); appetite loss; weight loss; taste changes; stomatitis and esophagitis (inflammation and sores); constipation; diarrhea; fatigue; heart damage; nervous system changes; lung damage; reproductive tissue damage; liver damage; kidney and urinary system damage.

It has been shown that cancer cell death can be immunogenic or non-immunogenic. Immunogenic cell death (ICD) comprises changes in the structure of the cell surface and leads to the release of pro-immunogenic factors. Subsequently it attracts APCs to take up tumor antigens, process them, and finally elicit antitumor immune response (specific anti-tumor T cells). The success of the cancer treatment whether using chemotherapy, oncolytic viruses or a combination of the two, relies on the induction of immunogenic tumor cell death and induction of anti-tumor immune responses. It has been known that some chemotherapeutics and oncolytic adenoviruses act as potent inducers of ICD, and thus have a beneficial impact on anticancer immune responses, contributing antitumor activity. ICD can be evaluated by the presence of ICD biomarkers such as calreticulin (CRT) in the outer plasma membrane, followed by extracellular release of high-mobility group box 1 protein (HMGB1) and adenosine triphosphate (ATP).

As disclosed earlier, cancer therapy with ONCOS-102 adenovirus as well as therapy with chemotherapeutics have shown some efficacy when used alone. The inventors wanted to study whether the combination of adenoviral gene therapy with other therapies, such as traditional therapy, may be more effective in the treatment of MM than either one alone.

"Combination therapy" as used herein refers to administration of ONCOS-102 and chemotherapeutic agents, preferably either Pemetrexed and Cisplatin or Pemetrexed and Carboplatin, to a patient in the need thereof. In the combination therapy the virus and also the chemotherapeutic agents may be administered in several doses during several days. The administration of chemotherapeutic agents can be started before starting virus administration and doses of chemotherapeutic agents can also be further administered during the period the virus doses are administered. The treatment protocol may also comprise first priming with the virus followed by administering the chemotherapeutic agents and then continuing administration of both the virus and chemotherapeutic agents. In another embodiment, the combination therapy first comprises chemotherapeutic agents Pemetrexed and Cisplatin, but during the combination therapy Cisplatin is replaced with Carboplatin. This can be necessitated for example because excessive side effects appear when Cisplatin used in the therapy. Also other medication can be administered concurrently with the combination therapy.

As used herein "concurrent" refers to a medication or therapy, which has been administered before, after or simultaneously with the combination therapy as described herein. The period for a concurrent therapy may vary from minutes to several weeks. Typically, the therapy that is concurrent with the combination therapy as described herein lasts for some days or some weeks.

The term "priming" as used herein refers to the using of apoptosis-inducing pretreatment with conventional cytotoxic chemotherapy capability. The tumor priming done with oncolytic viruses results in immunogenic cancer cell death, which is associated with the presentation of calreticulin on the cell surface and the release of natural adjuvants, specifically high-mobility group protein B1 (HMGB1) and ATP from within the dying cells, eventually leading to DC stimulation and subsequent activation of adaptive immune response. This virus-induced change in the tumor environment is essential in priming a meaningful antitumor immune response. Antigen presenting cells capture tumor antigens from dying tumor cells and process them for MHC class I and II presentation, migrate to draining lymph nodes and stimulate antigen-specific B and T cells. For example ONCOS-102 adenovirus may result in the induction of cytotoxic tumor-specific CD8+ T cells. The term "priming" as used herein refers also to the capability of chemotherapeutic agents - when administered before administering of the virus - to cause this kind of immunogenic cancer cell death. On the other hand tumor priming with chemotherapeutic agents also means expansion of interstitial space using an apoptosis-inducing pretreatment.

As used herein, the term "effective amount" refers to an amount of a compound, therapeutic agent, virus or drug, which is capable of performing the intended result. For example, an effective amount of a chemotherapeutic agent and/or adenovirus is an amount sufficient to effect beneficial or desired clinical results including clinical results. An effective amount can be administered in one or more administrations. As described herein, the effective amount is an amount sufficient to ameliorate, stabilize, reverse, slow and/or delay progression of malignant mesothelioma or to cure malignant mesothelioma. In the present invention, the effect of chemotherapeutic agents and ONCOS-102 adenovirus can be monitored for example by monitoring tumor reaction to said treatment. Monitoring of the tumor reaction may be performed with any suitable method known in the art. It can be performed for example with methods measuring immunologic status of the cells, such as those listed in Ranki et al. (2014). For example, monitoring can be done by measuring the presence of tumor infiltrating lymphocytes in the tumor, TH1-type response can be monitored with a microarray, and also IFNγ enzyme linked immunospot assay (ELISPOT) can be utilized in monitoring.

The effective amount is thus such that is capable to or required to cause a desired effect or reaction in the tumor. As is understood in the art, an effective amount may vary, depending on, inter alia, patient history as well as other factors such as the type and/or dosage of a chemotherapeutic agent used.

As used herein, the term "toxicity" refers to a toxic event associated with the administration of chemotherapeutic agents. Such events include, but are not limited to, neutropenia, thrombopenia, toxic death, fatigue, anorexia, nausea, skin rash, infection, diarrhea, mucositis, and anemia. The toxic effects can be monitored with any conventional method known in the field.

As used herein, the term "treatment period" refers to a time period when the combination treatment is conducted. Treatment period may consist of several administrations of ONCOS-102 and chemotherapeutic agents, wherein the administrations can be performed in cycles. Treatment period can last weeks or months. Treatment period can last up to one year.

As used herein, the term "administration period" refers to a time period the adenovirus, chemotherapeutic agents, or other medication are administered to a patient. During the administration period a single dose or several doses of the agent in question can be administered. The administration period can be minutes, hours, days, weeks or months. The administration period can consist of cycles. For example, a three-week (21-day) cycle can be used in the administration of chemotherapeutic agents.

Platinum-based antineoplastic drugs (informally called platins) are chemotherapeutic agents to treat cancer. They are coordination complexes of platinum. Carboplatin and cisplatin are platinum-based antineoplastic.

Pemetrexed is chemically similar to folic acid and is in the class of chemotherapy drugs called folate antimetabolites. Pemetrexed works by inhibiting three enzymes used in purine and pyrimidine synthesis―thymidylate synthase (TS), dihydrofolate reductase (DHFR), and glycinamide ribonucleotide formyltransferase (GARFT). By inhibiting the formation of precursor purine and pyrimidine nucleotides, pemetrexed prevents the formation of DNA and RNA, which are required for the growth and survival of both dividing normal cells and cancer cells.

Pemetrexed has been approved by FDA to be used in combination with Cisplatin for the treatment of unresectable MPM (500 mg/m² and 75 mg/m² respectively). Large I phase III EMPHACIS trial showed improved outcomes in patients treated with both Cisplatin and Pemetrexed compared to Cisplatin alone. It was also shown that the median survival of patients in the combination group was 12 months, compared to a median survival of 9 months in Cisplatin-only (p = 0.02). Due to high toxicity of Cisplatin in many MM patients, Carboplatin has been tested as an alternative. However, these first-line chemotherapeutics have all shown low efficacy against MM.

Therapeutic compositions are generally formulated relative to the particular administration route. In the combination therapy as described herein Pemetrexed is used in its effective concentration. As an example, for treatment of MM, Pemetrexed can be administered at a total daily dose of up to 500 mg/m². Pemetrexed can be administered for example as an intravenous (i.v.) infusion over 10 minutes on Day 1 of each 21-day cycle, when 21-day cycle is used. For example in case of side effects the dose can be lower, such as from 200 mg/m² to 450mg/m² and more preferably from 250 mg/m² to 375 mg/m². Pemetrexed can also be administrated intraperitoneally when appropriate.

In the combination therapy as described herein Cisplatin is used in its effective concentration. In the treatment of MM the typical dose of Cisplatin is 75 mg/m² infused i.v. over 2 hours beginning approximately 30 minutes after the end of Pemetrexed infusion. The amount of Cisplatin administered may vary from 30 mg/m² to 75 mg/m². For example in case of side effects the dose can be lower, such as from 30 mg/m² to 70 mg/m², more preferably from 35 mg/m² to 60 mg/m² and most preferably from 37.5 mg/m² to 56.25 mg/m². Cisplatin can also be administrated intraperitoneally when appropriate.

In the combination therapy with ONCOS-102 adenovirus Cisplatin and Pemetrexed can be administered in a molar ratio between from 0.75:10 to 3:10. Most preferably the ratio is 1.5:10.

In the combination therapy as described herein Carboplatin is used in its effective concentration. In the combination therapy treatment of MM the typical dose of Carboplatin is 400 mg/m² to a patient with normal renal function. Carboplatin can be infused i.v. over 30 to 60 min beginning approximately 30 minutes after the end of Pemetrexed infusion that has been infused over about 10 min time. However, the dose of Carboplatin is generally calculated according to Calvert formula (calculated in mg, not in mg/ m²) that takes into account a patient's glomerular filtration rate (GFR) and target area under the concentration versus time curve (AUC in mg/ml x min): Total dose (mg)=(target AUC) x (GFR + 25). Carboplatin dose can thus be adjusted and reduced for example due to side effects, especially due to reduced renal function.

In the combination therapy with ONCOS-102 adenovirus, the administrated doses are typically about 500 mg/m² of Pemetrexed and about AUC 5 of Carboplatin. In a preferred embodiment - and in case of patient having normal renal function - about 500 mg/m² Pemetrexed and about 400 mg/m² Carboplatin can be administered. The administered amounts can, however, vary between 250 mg/m² and 500 mg/m² of Pemetrexed and between 200 mg/m² and 400 mg/m² of Carboplatin. In certain cases, especially when the chemotherapeutic agents show toxic effects, about 375 mg/m² Pemetrexed and about 300 mg/m² Carboplatin can be administered. Pemetrexed and Carboplatin can thus be administered in a molar ratio of 5:4. The ratio can, however, vary between from 5:8 to 5:2.

In the combination therapy as described herein, ONCOS-102 adenovirus can be administered in several doses and at different times that chemotherapeutic agents that also can be administered in several doses.

An object of the present invention is ONCOS-102 adenovirus for use in the treatment of human malignant mesothelioma, wherein the virus is administered in combination with two chemotherapeutic agents to a patient, and wherein the two chemotherapeutic agents are either Pemetrexed and Cisplatin or Pemetrexed and Carboplatin.

Still another object of the present invention is ONCOS-102 adenovirus for use in the treatment of human malignant mesothelioma, wherein the virus is administered in combination with Pemetrexed and Cisplatin to a patient in need thereof, and wherein the molar ratio between Pemetrexed and Cisplatin is from 10:0.75 to 10:3.

Still another object of the present invention is ONCOS-102 adenovirus for use in the treatment of human malignant mesothelioma, wherein the virus is administered in combination with Pemetrexed and Cisplatin to a patient in need thereof, and wherein the molar ratio between Pemetrexed and Cisplatin is 10:1.5.

A further object of the present invention is ONCOS-102 adenovirus for use in the treatment of human malignant mesothelioma, wherein the virus is administered in combination with Pemetrexed and Carboplatin to a patient, wherein the molar ratio between Pemetrexed and Carboplatin is from 5:8 to 5:2.

Still another object of the present invention is ONCOS-102 adenovirus for use in the treatment of human malignant mesothelioma, wherein the virus is administered in combination with Pemetrexed and Carboplatin to a patient in need thereof, wherein the molar ratio between Pemetrexed and Carboplatin is 5:4.

In preferred embodiments of the present invention, various combinations of Cyclohexamide, Cyanocobolamine, Folatic acid and Dexamethasone can also be used in addition to ONCOS-102 and Pemetrexed and Cisplatin or Pemetrexed and Carboplatin. More specifically, from the list of agents consisting of Cyclohexamide, Cyanocobolamine, Folatic acid and Dexamethasone, one, two, three or four and any combination thereof can be used concurrently with the combination therapy comprising ONCOS-102 and Pemetrexed and Cisplatin or ONCOS-102 and Pemetrexed and Carboplatin. As an example, the subject can be treated with one or more adjunctive agents that reduce or eliminate hypersensitivity reactions before, during, and after administration of the chemotherapeutic agents of the combination therapy described, such as one or more of dexamethasone, folic acid, and Vitamin B12 before, during, and after administration of the chemotherapeutic agents. In certain embodiments, the subject is treated with 2-25 mg of dexamethasone orally on the day before, the day of, and the day after administration of the chemotherapeutic agents; 400-1000 µg of folic acid orally daily, during a period starting 7 days before administration of the chemotherapeutic agents, throughout at least one treatment period, and for 21 days after the last administration of the chemotherapeutic agents; and 1000 µg of Vitamin B12 intramuscularly 1 week before the first administration of the chemotherapeutic agents in a treatment period.

Any conventional method may be used for administration of ONCOS-102 to a patient in need thereof. The route of administration depends on the formulation or form of the composition, the disease, location of tumors, the patient, comorbidities and other factors. In a preferred embodiment of the invention, the administration is conducted through an intratumoral, intramuscular, intra-arterial, intrapleural, intravesicular, intracavitary or peritoneal injection, or an oral administration. Preferably, the administration is conducted as intratumoral injection or intraperitoneal injection.

The injection can be personalized for each patient according to the location and size of tumors. For example, the virus can be injected (i.t.) in a volume from 0.5 ml to 10 ml. The injection can be done to several, preferably up to five, different tumor sites. The volume of intraperitoneal dosage can vary from 200 ml to 800 ml. Preferably, the administered volume is 500 ml.

The effective dose of vectors depends on at least the subject in need of the treatment, tumor type, location of the tumor and stage of the tumor. The dose may vary for example from about 10⁸ viral particles (VP) to about 10¹⁴ VP, preferably from about 5×10⁹ VP to about 10¹³ VP and more preferably from about 8×10⁹ VP to about 10¹² VP. In one specific embodiment of the invention the dose is in the range of about 5×10¹⁰ - 5×10¹¹ VP. Thus, the amount of ONCOS-102 adenovirus to be administered can be in the range of 5×10¹⁰ - 5×10¹¹ VP. Preferably, ONCOS-102 is administered at 3 × 10¹¹ VP/5 ml. On the other hand, expressed in the plaque forming units, ONCOS-102 can be administered by direct injection into malignant mesothelioma tumor of said patient or intraperitoneally at a dose of about 10⁸-10¹² plaque forming units.

One object of the invention is ONCOS-102 adenovirus for use in the treatment of human malignant mesothelioma, wherein the virus is administered in combination with either Pemetrexed and Cisplatin, or Pemetrexed and Carboplatin to a patient in need thereof, and wherein the amount of the virus is from 5 × 10¹⁰ to 5 × 10¹¹ VP.

Another object of the invention is ONCOS-102 adenovirus for use in the treatment of human malignant mesothelioma, wherein the virus is administered in combination with either Pemetrexed and Cisplatin, or Pemetrexed and Carboplatin to a patient, and wherein the virus is administered in an amount of 3 × 10¹¹ VP/5 ml.

A further aspect of the invention is ONCOS-102 adenovirus for use in the treatment of human malignant mesothelioma wherein the virus is administered in combination with either Pemetrexed and Cisplatin, or Pemetrexed and Carboplatin to a patient, and wherein the chemotherapeutic agents and the virus are administered in effective amounts.

Still another object of the invention is ONCOS-102 adenovirus for use in the treatment of human malignant mesothelioma, wherein the virus is administered in combination with either Pemetrexed and Cisplatin, or Pemetrexed and Carboplatin to a patient in need thereof, and wherein the virus is administered intraperitoneally or by direct injection into tumor and the two chemotherapeutic agents are administered intravenously or intraperitoneally.

According to one aspect of the invention ONCOS-102 adenovirus is for use in the treatment of human malignant mesothelioma, wherein the virus is administered in combination with either Pemetrexed and Cisplatin, or Pemetrexed and Carboplatin to a patient in need thereof, and wherein the virus is administered before administering of the two chemotherapeutic agents and also during administering period of said chemotherapeutic agents.

According to another aspect of the invention ONCOS-102 adenovirus is for use in the treatment of human malignant mesothelioma, wherein either Pemetrexed and Cisplatin, or Pemetrexed and Carboplatin are administered before the virus is administered and also during administering period of said virus.

According to one preferred embodiment the agents of the combination therapy may be administered with first priming with administering the virus from one to ten times during a between one-month to ten-month period and then administering the chemotherapeutic agents for example from one to four weeks after starting the virus administration. The administration may for example be in the following sequential order: Priming with the virus is done by administering the virus to the subject in need six times during a four-month period. Typically, in the beginning of the treatment period the time between doses is shorter than when the treatment proceeds. The administration of

Pemetrexed and Cisplatin or Pemetrexed and Carboplatin, can be started after three weeks of starting the administration of the virus. The administration of chemotherapeutic agents can be continued so that the administration is for example done a total of six times in about three-week cycles. The administration times can vary from one to six times.

The oncolytic adenoviral vector disclosed herein induces virion-mediated oncolysis of tumor cells and activates human immune response against tumor cells. In a preferred embodiment the method or use further comprises administration of substances capable of downregulating regulatory T-cells in a subject. "Substances capable of downregulating regulatory T-cells" refers to agents that reduce the amount of cells identified as T-suppressor or Regulatory T-cells. These cells have been identified as consisting one or many of the following immunophenotypic markers: CD4+, CD25+, FoxP3+, CD127- and GITR+. Such agents reducing T-suppressor or Regulatory T-cells may be selected from a group consisting of anti-CD25 antibodies or chemotherapeutics.

The immunomodulatory functions of the transgene GM-CSF are a central mechanism of action of armed oncolytic adenoviruses and in addition, adenovirus itself is a strong activator of the immune system and this significantly contributes to the overall anti-tumor efficacy of the virus.

"Virus sensitizer" as used herein refers to an agent that can improve oncolytic virus efficacy. Agents suitable for such combination therapy or which can be used as virus sensitizers include but are not limited to All-trans retinoic acid, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, erlotinib, Etoposide, Fluorouracil, Gemcitabine, Hydroxyurea, Idarubicin, Imatinib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine. Preferably, the virus sensitizer to be used is cyclophosphamide.

Also check-point inhibitors, such as anti-PD-1/PD-L1 or anti-CTLA4 antibodies (such as Pembrolizumab, Nivolumab or Ipilimumab), are suitable for use with the combination therapy.

The present disclosure relates to a method for treating malignant mesothelioma in a patient comprising a step of administering to said patient ONCOS-102 adenovirus and two chemotherapeutic agents. ONCOS-102 adenovirus and two chemotherapeutic agents are administered in effective amounts.

The present disclosure relates to a method for treating malignant mesothelioma in a patient comprising a step of administering to said patient ONCOS-102 adenovirus and two chemotherapeutic agents in amounts and for a time sufficient to kill malignant mesothelioma cells or to prevent the growth of malignant mesothelioma cells.

The disclosure relates to a method for treating malignant mesothelioma in a patient comprising a step of administering to said patient ONCOS-102 adenovirus and two chemotherapeutic agents, wherein the chemotherapeutic agents are either Pemetrexed and Cisplatin or Pemetrexed and Carboplatin.

The disclosure relates to a method for treating malignant mesothelioma in a patient comprising the steps of administering to said patient ONCOS-102 adenovirus and two chemotherapeutic agents, wherein said chemotherapeutic agents are first administered before starting the administration period of the virus and also during the administration period of the virus.

The disclosure relates to a method for treating malignant mesothelioma in a patient comprising the steps of administering to said patient ONCOS-102 adenovirus and two chemotherapeutic agents, wherein said virus is first administered before starting the administration period of the chemotherapeutic agents and the virus is also administered during the administration period of said chemotherapeutic agents.

The disclosure relates to a method for treating malignant mesothelioma in a patient comprising the steps of administering to said patient ONCOS-102 adenovirus and two chemotherapeutic agents, wherein ONCOS-102 adenovirus is administered from one to ten times and the chemotherapeutic agents are administered from one to six times to said patient.

The disclosure relates to a method for treating malignant mesothelioma in a patient comprising the steps of administering to said patient ONCOS-102 adenovirus and two chemotherapeutic agents, wherein said administration of ONCOS-102 is done by direct injection into malignant mesothelioma tumor of said patient or as intraperitoneal injection at a dose of 10⁸-10¹² plaque forming units.

The disclosure relates to a method for treating malignant mesothelioma in a patient comprising the steps of administering to said patient ONCOS-102 adenovirus and two chemotherapeutic agents, wherein the amount of ONCOS-102 adenovirus to be administered is in the range of 5×10¹⁰ - 5×10¹¹ VP.

The disclosure relates to a method for treating malignant mesothelioma in a patient comprising the steps of administering to said patient ONCOS-102 adenovirus and two chemotherapeutic agents, wherein said chemotherapeutic agents are administered at doses from 250 mg/m² and 500 mg/m² of Pemetrexed and from 30 mg/m² to 75 mg/m² of Cisplatin or at doses from 250 mg/m² and 500 mg/m² of Pemetrexed and from 200 mg/m² to 400 mg/m² of Carboplatin.

The disclosure relates to a use of ONCOS-102 adenovirus in the treatment of human malignant mesothelioma, wherein the virus is administered to a patient in combination with two chemotherapeutic agents.

Concurrent administration of cyclophosphamide (CPO) to the patient can be done during the time period of combination therapy. Cyclophosphamide is a common chemotherapeutic agent, which has also been used in some autoimmune disorders. Cyclophosphamide has been shown to improve oncolytic virus efficacy through several mechanisms. It dampens the innate antiviral response, slows the generation of anti-oncolytic virus neutralizing antibodies, may target T-regs and may affect tumor vasculature enhancing oncolytic virus extravasation. Several preclinical studies have shown that cyclophosphamide can retard immune clearance of oncolytic viruses, enhance persistence of virus infection and prolong therapeutic efficacy. In the present invention, cyclophosphamide can be used as a virus sensitizer to enhance viral replication and the effects of GM-CSF induced stimulation of NK and cytotoxic T-cells for enhanced immune response against the tumor. It can be used as intravenous bolus doses or low-dose oral metronomic administration. Other suitable virus sensitizers that can be used in embodiments of the present invention include temozolomide and erlotinib.

To reduce regulatory T cells, the patients will receive low dose CPO, one to three days before each injection of ONCOS-102. CPO will be administered for example as an i.v. bolus of 300 mg/m². The bolus may vary between 100 and 600 mg/m². The route of administration of CPO can also be for example oral administration. Also metronomic chemotherapy may be used.

Also Folic acid can be administered to the patient before start of the combination therapy and also during the combination therapy. The administration of Folic acid can be started at least five days before administration of first dose of chemotherapeutic agents of the combination therapy. For example, the administration can be started 1 - 2 weeks before starting the administration of the chemotherapeutic agents of the combination therapy. Folic acid can be administered daily (oral administration; PO) and the administration can be continued also during the combination therapy. The administration can be continued until about three weeks after last dose of chemotherapeutic agents. The typical dose of Folic acid is 4 mg (PO). One preferred aspect of the invention is a method for treatment of mesothelioma in a patient in need thereof comprising the steps of (a) administering to said patient ONCOS-102 adenovirus and two chemotherapeutic agents and a step of administration of Folic acid to the patient. Cyanocobolamin (Vitamin B12) can be administered to the patient before start of the combination therapy and also during the combination therapy. The administration of Cyanocobolamin is typically started 1 - 2 weeks before starting the administration of the chemotherapeutic agents of the combination therapy. Cyanocobolamin can be administered for example as intramuscular injection (i.m.) on nine-week intervals and also during the combination therapy. The administration can be continued until about three weeks after last dose of chemotherapeutic agents. Typical amount of Cyanocobolamin is 1000 mcg (µg). One preferred aspect of the invention is a method for treatment of mesothelioma in a patient in need thereof comprising the steps of (a) administering to said patient ONCOS-102 adenovirus and two chemotherapeutic agents and a step of administration of Cyanocobolamin to the patient.

Folic acid and Cyanocobolamin are generally used to reduce treatment related hematologic and gastrointestinal toxicity. These side effects can be associated for example to the administration of Pemetrexed.

Also Dexamethasone can be administered to a patient subject to the combination therapy. Typically, Dexamethasone is administered day before, the day of and the day after administering of the chemotherapeutic agents of the combination therapy. Dexamethasone can be administered 4 mg BD (i.e. twice a day) for 5 days, and in a three-weekly frequency for up to six cycles. One object of the invention is a method for treatment of mesothelioma in a patient in need thereof comprising the steps of (a) administering to said patient ONCOS-102 adenovirus and two chemotherapeutic agents, and (b) administrating Dexamethasone to the patient.

The combination therapy as described herein can further comprise administration of cyclophosphamide to the patient. One object of the disclosure is a method for treatment of mesothelioma in a patient in need thereof comprising a step (a) of administering to said patient ONCOS-102 adenovirus and two chemotherapeutic agents and further comprising before step (a) administration of Cyclophosphamide to the patient.

The disclosure relates to a method for reducing tumor growth in a patient, wherein said method comprises administering ONCOS-102 adenovirus and two chemotherapeutic agents to said patient under conditions wherein tumor growth in said patient is reduced.

The method can comprise identifying the patient as having a tumor before administering the combination therapy. The tumor diagnosis can be done in any conventional method. The patient can be identified as having the tumor using for example a diagnostic imaging technique. The method can further comprise measuring a tumor growth reduction after administering the combination therapy to the patient. The tumor reduction can be studied by any conventional method. The tumor growth reduction can be measured for example using a diagnostic imaging technique.

The present disclosure relates to the use of combination therapy in order to inhibit the growth of tumor. Marks of the inhibition of the tumor growth may for example be a reduction in tumor weight and reduction in tumor volume. In addition, the combination therapy can be used to inhibit the spread of tumor.

It should be also noted that tumor pseudoprogression that corresponds to an increase of lesion size related to treatment, may affect the result of imaging follow-up intended to reveal the effect of the current combination therapy on the tumor size. This kind of effect has been observed after combined chemotherapy and radiotherapy in about 30% of patients. Pseudoprogression may thus be present in certain part of the patients when the combination therapy as described herein is used. For those patients reduction of tumor size is not a suitable indicator of the effectiveness of the therapy.

The disclosure relates to a method of reducing the growth of cancer cells comprising administering to a subject in need of treatment an effective amount of ONCOS-102 adenovirus and two chemotherapeutic agents.

The disclosure relates to a method of inhibiting or killing tumor or cancer cells in a human patient, consisting of treating the patient with ONCOS-102 adenovirus and two chemotherapeutic agents, wherein the chemotherapeutic agents are either Pemetrexed and Cisplatin or Pemetrexed and Carboplatin.

Further, the disclosure relates to a method of killing tumor or cancer cells comprising contacting the tumor or cancer cells with ONCOS-102 adenovirus and two chemotherapeutic agents, wherein the chemotherapeutic agents are either Pemetrexed and Cisplatin or Pemetrexed and Carboplatin.

Further, the disclosure also relates to a method for treating malignant mesothelioma comprising administering to a subject in need thereof an effective amount of ONCOS-102 adenovirus and effective amounts of two chemotherapeutic agents to provide a combination therapy having an enhanced therapeutic effect compared to the effect of the ONCOS-102 adenovirus administered alone or of the two chemotherapeutic agents administered together without said virus.

In an embodiment of the invention, ONCOS-102 acts as an *in situ* cancer vaccine. As used herein *"in situ* cancer vaccine" refers to a cancer vaccine, which both kills tumor cells and also increases the immune response against tumor cells. Virus replication is a strong danger signal to the immune system (=needed for a TH1 type response), and thus acts as a powerful costimulatory phenomenon to GM-CSF mediated maturation and activation of APCs, and recruitment of NK cells. Tumor cell lysis also helps to present tumor fragments and epitopes to APCs and furthermore, costimulation is produced by inflammation. Thus, an epitope independent (i.e. not HLA restricted) response is produced in the context of each tumor and therefore takes place *in situ.* Tumor specific immune response is activated in the tumor environment when specific tumor antigens are released from dying cells upon tumor cell lysis.

In a preferred embodiment of the disclosure the method or use further comprises administration of concurrent radiotherapy to a subject.

In a preferred embodiment of the disclosure the method or use further comprises administration of autophagy inducing agents to a subject. Autophagy refers to a catabolic process involving the degradation of a cell's own components through the lysosomal machinery. "Autophagy inducing agents" refer to agents capable of inducing autophagy and may be selected from a group consisting of, but not limited to, mTOR inhibitors, PI3K inhibitors, lithium, tamoxifen, chloroquine, bafilomycin, temsirolimus, sirolimus and temozolomide. In a specific embodiment of the invention, the method further comprises administration of temozolomide to a subject. Temozolomide may be either oral or intravenous temozolomide.

An object of the present disclosure was to develop novel therapeutically effective use of ONCOS-102 oncolytic adenovirus and chemotherapeutic agents with improved safety properties and with improved effect on cancer as compared to using viral therapy alone or chemotherapy only.

The results of the current experiments showed that ONCOS-102 as a monotherapy killed human mesothelioma cell lines *in vitro* and showed some anti-tumor activity in treatment refractory H226 malignant pleural mesothelioma xenograft model. On the contrary, current standard of care (SoC) chemotherapy regimens for malignant mesothelioma (Pemetrexed + Cisplatin or Pemetrexed + Carboplatin) showed no anti-tumor efficacy in this mesothelioma xenograft model. However, surprisingly a synergistic anti-tumor effect was seen when ONCOS-102 was combined with SoC chemotherapy regimens. This data give a strong rationale for combining ONCOS-102 with SoC chemotherapy for the treatment of malignant mesothelioma.

Chemotherapy - when administered with ONCOS-102 - increased the expression of ICD marker in the current xenograft model. It has earlier been shown that ONCOS-102 produces functional human GM-CSF in hamsters, inducing tumor-specific immunity indicating that GMCSF might be involved in the recruitment and activation of dendritic cells (DC) also in the current xenograft model, leading to stimulation of T-cells and finally to the development of anti-tumor immunity. In the human mesothelioma cancer model described in details in the examples, function of GM-CSF was lost due to immunodeficient murine immune system. However, GM-CSF utility on immune system modulation against mesothelioma has been already shown in clinical studies (Ranki et al. 2014).

Previous clinical data has demonstrated that ONCOS-102 is able to prime a tumor specific immune response, meaning induction of cytotoxic tumor-specific CD8+ T cells, in chemotherapy refractory malignant pleural mesothelioma patient (Ranki et al. 2014). However, in this current immunodeficient animal model, priming with ONCOS-102 before initiation of chemotherapy did not show any additional benefits. This is not surprising since the immune system plays a very minimal role in the immunodeficient mouse model.

The current data strongly suggest that ONCOS-102 combined with first-line chemotherapy can be used as an efficient treatment against MM. Due to its safety profile, ONCOS-102 was replicating only locally in tumors and exhibited high tropism to mesothelioma cells. No major side effects were reported during the animal study. Combined treatment showed synergistic effect and was the most effective therapy against mesothelioma and was thus also more effective than standard first-line chemotherapy. Additionally, combination of ONCOS-102 with chemotherapy is a powerful tool in order to overcome the major obstacle of immune suppressive microenvironment in tumor microenvironment due to enhanced induction of immunogenic tumor cell death and finally induction of anti-cancer immune responses. The inventors have shown that combined treatment leads to an increase of ICD, suggesting enhanced activity of tumor immunogenic cell death. Additionally, combination treatment improved infectivity of ONCOS-102 in mesothelioma cells, GM-CSF function in this animal model was lost, but it is known from previous studies that this cytokine plays an important function by directly recruiting APCs and natural killer cells, as well as by activating and maturing APCs at the tumor site.

Besides enabling the transport of the vector to the site of interest the adenovirus vector of the invention also assures the expression and persistence of the transgene. Furthermore, immune response against the vector as well as the transgene is minimized.

The present invention solves problems related to therapeutic resistance to conventional treatments. Furthermore, the present invention provides tools for selective treatments, with less toxicity or damages in healthy tissues. Advantages of the present invention include also different and reduced side effects in comparison to other therapeutics. Importantly, the approach is synergistic with many other forms of therapy including radiation therapy, and is therefore suitable for use in combination regimens.

The present invention achieves cancer therapy, wherein tumor cells are destroyed by virion-caused oncolysis. In addition, various different mechanisms activating human immune response, including activation of natural killer cells (NK) and dendritic cells (DC) are recruited for therapeutic use in the present invention.

This application thus describes strategies and provides methods and means to both effectively recruit the host's immune system against malignant cells and simultaneously provide direct oncolytic and chemotherapeutic activity in malignant cells, while maintaining an excellent safety record.

Aspects of the disclosure are directed to novel methods and means for achieving efficient and accurate gene transfer as well as increased specificity and sufficient tumor killing ability in cancer therapy.

The unexpected efficacy of the inventive therapy with oncolytic adenovirus combined with the use of chemotherapeutic agents provides a significant improvement in the therapeutic efficacy as demonstrated in *in vivo* studies in comparison to current standard of care (SoC) chemotherapy regimens for malignant mesothelioma (Pemetrexed + Cisplatin or Pemetrexed + Carboplatin).

In summary, presented data give a strong rationale for combining ONCOS-102 with first-line chemotherapy for the treatment of malignant mesothelioma.

### EXAMPLES

The following examples are given solely for the purpose of illustrating various embodiments of the invention and they are not meant to limit the present invention.

### Construction of ONCOS-102

The construction and characterization of chimeric oncolytic adenovirus coding for human GM-CSF (ONCOS-102) has been described previously (WO 2010/072900 and Koski et al.) Shortly, Ad5/3-D24-GMCSF was generated and amplified using standard adenovirus preparation techniques. A pAdEasy-1-derived plasmid containing a chimeric 5/3 fiber, pAdEasy5/3, was created by homologous recombination in *Escherichia coli* of Ad5/3luc1 viral genome and BstXI-digested 8.9 kb fragment of pAdEasy-1. Next, a shuttle vector containing a 24-bp deletion in ElA(pShuttleD24) was linearized with *PmeI* and recombined with pAdEasy5/3 resulting in pAd5/3-D24. In order to insert human GMCSF gene into E3 region, an E3-cloning vector pTHSN was created by inserting SpeI to NdeI fragment from Ad5 genome into the multicloning site of pGEM5Zf⁺ (Promega, Madison, WI). pTHSN was further digested with *Sun*I/*Mun*I creating a 965-bp deletion in E3 region (*6.7K* and *gpl9K* deleted). The 432 bp complementary DNA encoding human GM-CSF (Invitrogen, Carlsbad, CA) was amplified with primers featuring specific restriction sites *Sun*I/*Mun*I flanking the gene and then inserted intoSunI/MunI-digested pTHSN. pAd5/3-D24-GMCSF was generated by homologous recombination in *E. coli* between FspI-linearized pTHSN-GMCSF and *Srf*I-linearized pAd5/3-D24. Ad5/3-D24-GMCSF virus genome was released by *Pac*I digestion and transfection to A549 cells for amplification and rescue. All phases of the cloning were confirmed with PCR and multiple restriction digestions. The shuttle plasmid pTHSN-GM-CSF was sequenced. Absence of wild-type E1 was confirmed with PCR. The E1 region, transgene, and fiber were checked in the final virus with sequencing and PCR. Virus production was done, according to the principles of cGMP by Oncos Therapeutics (Helsinki, Finland), on A549 cells to avoid the risk of wild-type recombination. Virus stock buffer formulation was 10 mmol/l Trizma base, 75 mmol/l NaCl 5% (wt/vol) sucrose, 1 mmol/l MgCI, 10 mmol/l _{L}(+)histidine, 0.5% (vol/vol) EtOH, 0.02% Tween, 100 µmol/l EDTA; 0.9% (wt/vol) NaCl solution (B. Braun, Melsungen, Germany) was used as a diluent. ONCOS-102 was produced by Biovian (Turku, Finland) in GLP and stored at -80°C until use. For use, ONCOS-102 was thawed shortly before application and stored on ice until use. After thawing, ONCOS-102 was diluted in laminar flow box to obtain the required concentration, injection syringes were prepared and stored on ice until use.

### Cell lines

Human epithelioid mesothelioma cell line JL-1 (ACC 596, purchased from DSMZ) was cultured in Dulbecco's modified Eagle medium (DMEM, 10567-014, Gibco) supplemented with 20% heat-inactivated fetal bovine serum (FBS, 16000-044, Gibco), 2 mM L-glutamine, 1% penicillin & streptomycin (15140-122, Gibco). Human malignant biphasic mesothelioma MSTO-211H (ACC 390, purchased from DSMZ) and human epithelial mesothelioma NCI-H226 (H226, CRL-5826, purchased from ATCC) were cultured in RPMI 1640 (A10491-01, Gibco) supplemented with 10% heat-inactivated FBS (16000-044, Gibco), 2 mM L-glutamine, 1% penicillin & streptomycin (15140-122, Gibco). All cell lines were incubated at 37°C with 5% CO₂.

### Chemotherapeutic agents

Pemetrexed Disodium (sc-219564), Cisplatin (sc-200896) and Carboplatin (sc-202093) were all purchased from Santa Cruz Biotechnology and reconstituted in sterile water before use.

10 mg of Pemetrexed (pem) was suspended into 1 ml of sterile water to obtain a 10 mg/ml solution. This solution was used as a stock solution and stored at room temperature. The substance was shaken from 1 minute up to 5 minutes upon addition of the solution to get a homogenous solution. The stock solution was further diluted in 0.9% NaCl and 10 mg/kg/mouse in 100 µl was used.

150 mg of Cisplatin (cis) was suspended into 150 ml of sterile water to obtain a 1 mg/ml solution. This solution was used as a stock solution and stored at room temperature. The substance was shaken from 1 minute up to 5 minutes upon addition of thesolution to get a homogenous solution. The stock solution was further diluted in 0.9% NaCl and 1.5 mg/kg/mouse in 100 µl was used.

25 mg of Carboplatin (car) was suspended into 10 ml of 0.9% NaCl to obtain a 2.5 mg/ml solution. This solution was used as a stock solution and stored at room temperature. The substance was shaken from 1 minute up to 5 minutes upon addition of the solution to get a homogenous solution. The stock solution was further diluted and 8mg/kg/mouse in 100 µl was used.

### Cell viability - in vitro tumor cell killing assay

Mesothelioma cells were seeded at 1.0 × 10⁴ cells per well on 96-well plates. After overnight incubation the cells were infected with ONCOS-102 with a viral particles/cell ratio of 10 (VP/cell). The virus and chemotherapeutic agents were diluted in media containing 5% FBS. Pemetrexed, Cisplatin and Carboplatin were tested at the following sub-optimal, previously selected concentrations 0.625 mg/ml, 0.0026 mg/ml, 0.0625 mg/ml (H226 cells); 0.625 mg/ml, 0.0006 mg/ml, 0.0019 6mg/ml (JI-1 cells); 0.083 mg/ml, 0.0026 mg/ml, 0.0625 mg/ml (MSTO-211H cells), respectively. Eight different treatment combinations were evaluated: ONCOS-102 alone, Pemetrexed + Cisplatin, Pemetrexed + Carboplatin, ONCOS-102 + Pemetrexed + Cisplatin, ONCOS-102 + Pemetrexed + Carboplatin, ONCOS-102 + Pemetrexed + Cisplatin (priming: virus administered first, chemotherapy added 24 hours post infection), ONCOS-102 + Pemetrexed + Carboplatin (priming: virus administered first, chemotherapy added 24 hours post infection) and mock (growth media only). Cell viability was determined 3 days later by MTS according to the manufacturer's instructions (G3582, Promega).

### Analysis of apoptotic and necrotic cells in vitro

Mesothelioma cells were seeded onto 6 well plates at 5×10⁵ cells/well. Cells were infected with 10 VP/cell of ONCOS-102 supplemented with chemotherapeutics according to the treatment scheme mentioned above. The amount of apoptotic and necrotic cells were measured 48 hours later with a TACS Annexin V-FITC kit by flow cytometer (4830-01-K, Trevigen Inc.) according to the manufacturer's instructions.

### Immunogenicity of tumor cell death in vitro

**CRT exposure.** Cell lines were seeded in duplicate onto 6 well plates at 5×10⁵ cells/well. Cells were infected with 10 VP/cell of ONCOS-102 and/or with chemotherapeutic agents according to the treatment combinations presented above. 24 (H226, JI-1) and 48 (MSTO-211H) hours later cells were harvested and stained with 1:1000 diluted rabbit polyclonal anti-Calreticulin antibody (ab2907, Abcam) for 40 min at 4°C and subsequently with 1:100 diluted Alexa-Fluor 488 secondary antibody (A21206, Invitrogen) and analyzed by flow cytometry.

**HMGB-1 release.** Cell lines were seeded in triplicates onto cell culture plates and infected with 10 VP/cell of ONCOS-102 and/or with chemotherapeutic agents according to the treatment combinations presented above. 72 hours later, supernatants were collected and HMGB-1 was measured with an Elisa kit according to manufacturer's instruction (ST51011, IBL International).

**ATP release.** Cell lines were seeded in triplicate onto cell culture plates and treated as mentioned above. Supernatants were collected after 48 (Jl-1, MSTO-211H) and 72 (H226) hours and analyzed with ATP Determination Kit according to manufacturer's protocol (A22066, Invitrogen) for luminometric analysis.

### Virus infectivity in vitro - immunocytochemistry assay (ICC)

Mesothelioma cell lines were seeded in 5 replicates onto 24 well plates at 6x105 cells/well and treated with eight different treatment combinations mentioned above. 24 hours later, supernatant was aspirated and cells were fixed by incubation with ice-cold methanol for 15 minutes (322415-100ML, Sigma-Aldrich). The determination of the ONCOS-102 infectivity was based on visual quantification of viral hexon protein in infected cells. Briefly, cells were stained with 1:2000 diluted mouse anti-hexon antibody (NB600-413, Novus Biologicals) for 1 hour at RT in the dark and subsequently with 1:500 diluted Biotin-SP-conjugated secondary antibody (115-065-062, Jackson Immuno Research) for 1 hour at RT in the dark. Subsequently the Extravidin-peroxidase was added at 1:200 and incubated for 30 minutes at RT in the dark (E2886-1ML, Sigma-Aldrich). Finally, the infected cells were visualized by adding the stain: DAB up to 5 minutes (D3939-1SET, Sigma-Aldrich). For each 5 replicates (wells) 5 images of non-overlapping fields was acquired using an AMG EVO XL microscope. Infectivity data is presented as the average number of spots in 5 wells.

### CAR, CD46, and DSG2 expression in mesothelioma cell lines

Human adenoviruses infect cells upon attaching to Coxsackie virus B and Ad receptor (CAR), CD46 or desmoglein 2 (DSG-2). Mesothelioma cells were labeled with mouse monoclonal anti-CAR antibody (1µg/1×10⁶cells), (sc-56892 FITC, Santa Cruz Biotech) for 40 min at 4°C, or with mouse monoclonal anti-CD46 antibody (1µg/1×10⁶cells) (ab789, Abcam) or mouse monoclonal anti-DSG2 antibody (1µg/1×10⁶cells) (ab14415, Abcam) for 40 min at 4°C and subsequently with 1:2000 diluted Alexa-Fluor 488 secondary antibody (a21206, Abcam) for flow cytometric measurements.

### Justification of dose level and route

The immunomodulatory functions of the transgene GM-CSF are a central mechanism of action of ONCOS-102. In addition, adenovirus itself is a strong activator of innate immune system. BALB/c nude mice are immunodeficient and thus only replication-dependent oncolytic function of the virus can be studied, and thereby a major mechanism (immune mediated effects) of action is lost. The dose 5E+7 VP/tumor i.t. (1E+8 VP/mouse) was chosen based on results of two pilot studies where doses 2E+8/mouse and 1E+8/mouse were tested (Figures 5 and 6). Suboptimal viral dose (=not curative) was selected to allow evaluation of potentially additive/synergistic combinatorial effects of ONCOS-102 and chemotherapy. The injection route was chosen based on the route used in clinical trials, i.e. intratumoral.

The dose and route of administration of chemotherapeutics were chosen based on technical feasibility.

### Animal selection, randomization, group assignment, housing, diet and water and acclimation

Animals were sorted randomly in to the groups. Animal groups were kept in separate cages with study cards attached to the cages. The cards were marked with the study number, treatment group, individual animal IDs, as well as their origin and date of arrival. The individual animals in each cage were marked with ear piercing for identification. Only one cage was handled at a time to avoid mixing the animals between the cages.

The animals were housed in biosafety level 2 (BSL2) facility in humidity- and temperature monitored environment. Animals were housed in individually ventilated plastic cages with a filter lid (GreenLine, Scanbur).

The animals fed standard pelletized diet provided *ad libitum.* Water was supplied *ad libitum* during the acclimation and study periods.

Animals were acclimated for 7 days prior to starting the experimental part. All the animals remained in good health throughout the acclimation period.

### Clinical observations

All animals were observed for clinical signs, morbidity or mortality daily during acclimation and administration period and additionally 60 min after each treatment. In the course of the experiment, the mice were killed due to tumor dimensions reaching the maximum allowed (1.5 cm in any diameter). The health of the animals was followed daily and no indication of discomfort due to the tumors was seen. Mice were killed with carbon dioxide on day 63, which was the end point of the experiment. In addition, a cervical dislocation was done.

The weight of the animals was followed throughout the experiment, and a minor weight loss was observed in the following groups: pem+cis (10% of decrease at day 48, 11% at day 60), ONCOS-102 priming+ pem + cis (9% of decrease day 48, 12% at day 60) (Figure 5B), pem+car (6% at day 60) (Figure 6B). Few animals were sacrificed due to health problems (>30% loss of body weight and skin problems (=rash) in groups treated with either ONCOS-102 alone or in combination with pemetrexed and cisplatin (single case). These adverse events were likely not related to the treatment as also two mock treated animals suffered from the skin problem (rash). Veterinarian was notified of these issues for further investigation but cause for these events remained unclear. In addition, necropsy showed some changes in morphology of intestines (jejunum and ileum) and stomach in few animals (pem+ cis; pem+ car).

### Analysis of tumor size progression

Tumor sizes were measured and recorded starting 8 days after the cell injections. The first day of treatment and first day of tumor size measurement are indicated as day 0 in Figure 4A. Tumors were measured every three days.

The longest and shortest diameter were recorded and the tumor volume was calculated using a formula of 0.52 × length × (width)². The tumor size progression was indicated as percentage of the first measurement point on day 0 which was arbitrarily set as 100 %.

### Data compilation and statistical analysis

**Body weight** was measured every 3 days throughout the whole experiment. Percentage body weight change from baseline was calculated for each animal.

**Tumor volumes** (Table 2): An absolute tumor volume was the end point in tumor volume analysis. This was assessed separately at days 21 and 48. Analysis of variance for repeated measurements was applied as analysis method for tumor volumes as there were two tumors measured for each mouse. The model contained the group effect and the baseline value as covariate as fixed effects. Pair-wise comparisons between groups were adjusted with Tukey's method. Synergism calculation was done by using FTV (fractional tumor volume) method.

Rationale for selection of time points: Day 21 was the last day when all animals were still alive, and on day 48 no more than one animal had died per group.

### Human mesothelioma xenograft model

Animal experiments were approved by the Experimental Animal Committee of the University of Helsinki and the Provincial Government of Southern Finland. 6-8 weeks old mice were obtained from Scanbur (Karlslunde, DK) and quarantined for 1 week before the experiment started. Mice were anesthetized with isoflurane Forane (Baxter), NCI-H226 cells in 50 µl were injected into both flanks (6E+06/flank). Tumors were let to grow 8 days prior to the treatments. Viruses were administered every 6 days. One group received ONCOS-102 only, two groups received ONCOS-102 and chemotherapy (Pemetrexed+Cisplatin or Pemetrexed+Carboplatin) every six days while two other groups received ONCOS-102 priming followed by combinatorial treatment of chemotherapy (pemetrexed+cisplatin or pemetrexed+carboplatin) and ONCOS-102 in 3-day cycles by turns. Mock animals were treated with 0.9 % saline. ONCOS-102 was diluted into 0.9 % saline and injected intratumorally at a dose of 5×10⁷ VP per tumor (two tumors per animal). Injections were given in a fan-like pattern to ensure even distribution throughout the tumor. Pemetrexed, Cisplatin, and Carboplatin were diluted in 0.9% NaCl and administrated intraperitoneally at doses of 10 mg/kg, 1.5 mg/kg, and 8 mg/kg, respectively. The injection volume was 100 µl per chemotherapeutic agent.

Tumor size was measured with caliper on two dimensions every 3 days, starting on the first treatment day. The longest and shortest diameter were recorded and the tumor volume was calculated using a formula of 0.52 × length × (width)². The tumor size progression was indicated as percentage of the first measurement point on day 0 which was arbitrarily set as 100 % (Figure 4A). All animals were observed for clinical signs, morbidity or mortality daily during acclimation and administration period and additionally 30 minutes after each treatment. During the experiment, the mice were killed when tumor dimensions reached the maximum allowed (1.5 cm) diameter.

### Human GM-CSF ELISA

Total protein from harvested BALB/c nude tissue samples (tumor and liver) were extracted using Tissue Extraction Reagent I (FNN0071, Invitrogen) supplemented with protease inhibitor cocktail (P-2714, Sigma-Aldrich) according to manufacturer's instruction. Protein extractions and previously collected serum were analyzed for human GM-CSF concentration using ELISA (ab100529, Abcam) according to manufacturer's instruction.

### Quantitative real-time PCR

qPCR for adenovirus E4 copy number was carried out according to the protocol previously described (primer FW: 5'-GGA GTG CGC CGA GAC AAC-3', primer RV: 5'-ACT ACG TCC GGC GTT CCA T-3', probe E4: 5'-(6FAM)-TGG CAT GAC ACT ACG ACC AAC ACG ATC T-(TAMRA)-3') (Koski et al. 2010). Total DNA was extracted from BALB/c nude murine samples (tumors, livers, blood) using the QIAamp DNA Blood Mini Kit (51106, Qiagen) according to manufacturer's protocol. Subsequently, the isolated DNA was analyzed for adenoviral E4 copy number that was normalized to murine beta-actin (liver, blood) and human beta-actin (tumor), respectively (primer FW: 5'-CGA GCG GTT CCG ATG C-3', primer RV: 5'-TGG ATG CCA CAG GAT TCC AT-3', probe murine beta-actin: 5'-(6FAM)-AGG CTC TTT TCC AGC CTT CCT TCT TGG-(TAMRA)-3'; (primer FW: 5'-CAG CAG ATG TGG ATC AGC AAG-3', primer RV: 5'-CTA GAA GCA TTT GCG GTG GAC-3', probe human beta-actin: 5'-(6FAM)- AGG AGT ATG ACG CCG GCC CCT C-(TAMRA)-3'). Samples were analyzed using LighCycler qPCR machine (LighCycler 480, Roche).

### Statistical analysis

Statistical significance was analyzed using one-way ANOVA with Tukey's Multiple Comparison test and nonparametric Mann-Whitney test. Survival curves and the statistical analysis were performed using Kaplan-Meier test. All statistical analysis, calculations and tests were performed using GraphPad Prism 5 (GraphPad Software, San Diego, USA). Results are presented as mean±SEM. All p values were two-sided or one-sided and considered statistically significant when ≤ 0.05.

### Example 1. Improved antineoplastic activity in vitro of ONCOS-102 combined with first-line chemotherapy against mesothelioma

Oncolytic potency of ONCOS-102 was tested in three mesothelioma cell lines *in vitro* (Fig. 1A). JL-1 (epithelioid mesothelioma), MSTO-211H (malignant biphasic) and H226 (epithelial morphology) cells appeared to be relatively resistant to oncolysis as 10 VP/cell (sub-optimal dose) killed 18%, 24% and 11% of cells respectively in 3 days. JL-1 and H226 cell lines were more resistant to chemotherapy-mediated cytotoxicity compared to MSTO-211H cells (Pemetrexed + Cisplatin or Pemetrexed + Carboplatin). Incubation with chemotherapeutics only killed 10% of JL-1 and 11-12% of H226 cells in 3 days. In contrast, 63% and 73% of MSTO-211H cells were killed by day 3 in co-culture with Pemetrexed + Cisplatin and Pemetrexed + Carboplatin, respectively. Compared to results observed in the single-treatment (virus or chemotherapy), the combination of ONCOS-102 with chemotherapeutics significantly increased cytotoxicity in H226 and JL-1 cells. However, no increased cytotoxicity was seen in MSTO-211H cells when chemotherapy was combined with ONCOS-102. In general, H226 and JL-1 cells were more resistant to both oncolysis (ONCOS-102 alone), and cytotoxic effect of chemotherapeutics than MSTO-211H cells which were particularly sensitive to chemotherapy.

In line with the cell viability results, the number of apoptotic H226 and JL-1 cells were generally low in all treatment groups, but a combination treatment slightly increased the number of apoptotic cells in comparison to monotherapies. In contrast to H226 and JL-1 cells, the number of apoptotic MSTO-211H cells were significantly higher in chemotherapy alone and chemotherapy+ONCOS-102 treated cells (Fig. 1B).

### EXAMPLE 2. Combination of ONCOS-102 and chemotherapy enhances immunogenic cell death and viral replication in mesothelioma cell lines

Immunogenic cell death markers such as an exposure of calreticulin A to cell surface and an extracellular release of ATP and HMGB1 were measured from mesotheolima cell cultures after exposure to ONCOS-102, chemotherapeutic agents, or combination of both (Figure 2). The most immunogenic tumor cell death in JI-1 and H226 mesothelioma was induced by treatment with ONCOS-102 + chemotherapy as the highest amount of CRT exposure and extracellular release of HMGB1 and ATP was measured in these groups. In turn simultaneous application of ONCOS-102 and chemotherapy was as immunogenic as chemotherapy alone in MSTO-211H cells, which is in line with the observations from cell viability assays and confirms the high sensitivity of these cells to chemotherapy.

The impact of chemotherapy on ONCOS-102 replication *in vitro* was also assessed. H226 and JI-1 cells infected with ONCOS-102 and pre-treated (primed) with chemotherapeutic agents (Pemetrexed + Cisplatin or Pemetrexed + Carboplatin) showed a significant increase in the number of infected cells over the control cells (p<0.001 vs. ONCOS-102) (Figure 3C). Pretreatment of MSTO-211H cells with chemotherapy, on the other hand, did not increase the number of infected cells. In contrast, chemotherapy administered simultaneously with ONCOS-102 into three mesothelioma cell lines lead to the inhibition or limitation of virus replication *in vitro.*

### EXAMPLE 3. ONCOS-102 displays high tropism for mesothelioma cells

ONCOS-102 is a chimeric oncolytic adenovirus, where fiber knob 5 was replaced by a serotype knob 3 domain. Positive cells for CD46, DSG2 (Ad3), and CAR (Ad5) receptor were screened (Fig. 3B). MSTO-211H, H226 and JL-1 expressed high level of CD46 (98%, 96% and 98% respectively), DSG2 (95%, 7% and 64% respectively) on their surfaces. The CAR receptor was expressed by two out three mesotheliomas: H226 (88%) and JI-1 (15%).

### EXAMPLE 4. ONCOS-102 in combination with Pemetrexed and Cisplatin or Pemetrexed and Carboplatin showed improved antineoplastic efficacy against MM in human xenograft mesothelioma model

Given the observed synergy of ONCOS-102 and Pemetrexed and Cisplatin or Pemetrexed and Carboplatin *in vitro* it was next assessed whether there exists also an enhanced anti-tumor activity in an *in vivo* animal model. Therefore human mesothelioma H226 cell line was subcutaneously implanted in BALB/c nude mice and tumors were treated according to the treatment regime presented in Table 1.

Tumor growth (volume) was calculated at indicated time points and reported as a function of time (Figure 4A). H226 mesothelioma xenograft tumors appeared to be refractory against standard chemotherapeutics (Pemetrexed+Cisplatin, Pemetrexed+Carboplatin), as none of the treatments significantly reduced tumor growth. Chemotherapy alone was the most inefficient treatment modality against mesothelioma due in part to the suboptimal dose used. One animal treated with ONCOS-102 + Pemetrexed + Cisplatin showed a complete tumor regression (both tumors) by day 21. In addition, one animal treated with ONCOS-102 priming + Pemetrexed + Cisplatin showed a complete regression of both tumors by day 45. ONCOS-102 alone was able to control tumor growth to some degree (p=0.074 mock vs. ONCOS-102 on day 48). The most effective treatment modality against pleural mesothelioma was with Pemetrexed and Cisplatin primed with ONCOS-102 (this treatment showed 97 % of initial tumor size at day 60 vs. 473% (mock), 563% (Pemetrexed + Cisplatin) and 672 % (Pemetrexed and Carboplatin). Additionally in all combination regiments (ONCOS-102 + chemotherapy) we observed the most significant antineoplastic activity especially compared with chemotherapy alone, virus alone and mock (initial tumor size: 97 % (virus priming + Pemetrexed + Cisplatin), 138% (virus + Pemetrexed + Cisplatin), 151 % (virus priming + Pemetrexed + Carboplatin) and 163 % (virus + Pemetrexed + Carboplatin) vs. 206 % (virus alone), 473 % (mock), 563 % (Pemetrexed + Cisplatin) and 672% (Pemetrexed + Cisplatin) at day 60). ONCOS-102 and chemotherapeutics, in all tested combinations, showed a strong synergistic anti-tumor effect (R=2.19 ONCOS-102 + Pemetrexed + Cisplatin; R=1.7 ONCOS-102 + Pemetrexed + Carboplatin; R=2.5 ONCOS-102 priming + Pemetrexed + Cisplatin; R=1.41 ONCOS-102 priming + Pemetrexed + Carboplatin) on day 48. On day 21, ONCOS-102 combination with Pemetrexed + Cisplatin showed synergistic effect on both regimens (R=1.8 ONCOS-102 + Pemetrexed + Cisplatin; R=1.32 ONCOS-102 priming + Pemetrexed + Cisplatin) while combination with Pemetrexed + Carboplatin showed an additive effect (R=1.0 and 0.93 for combination groups).

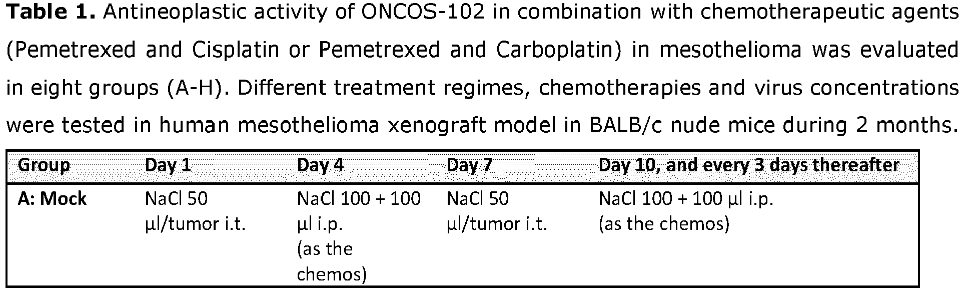

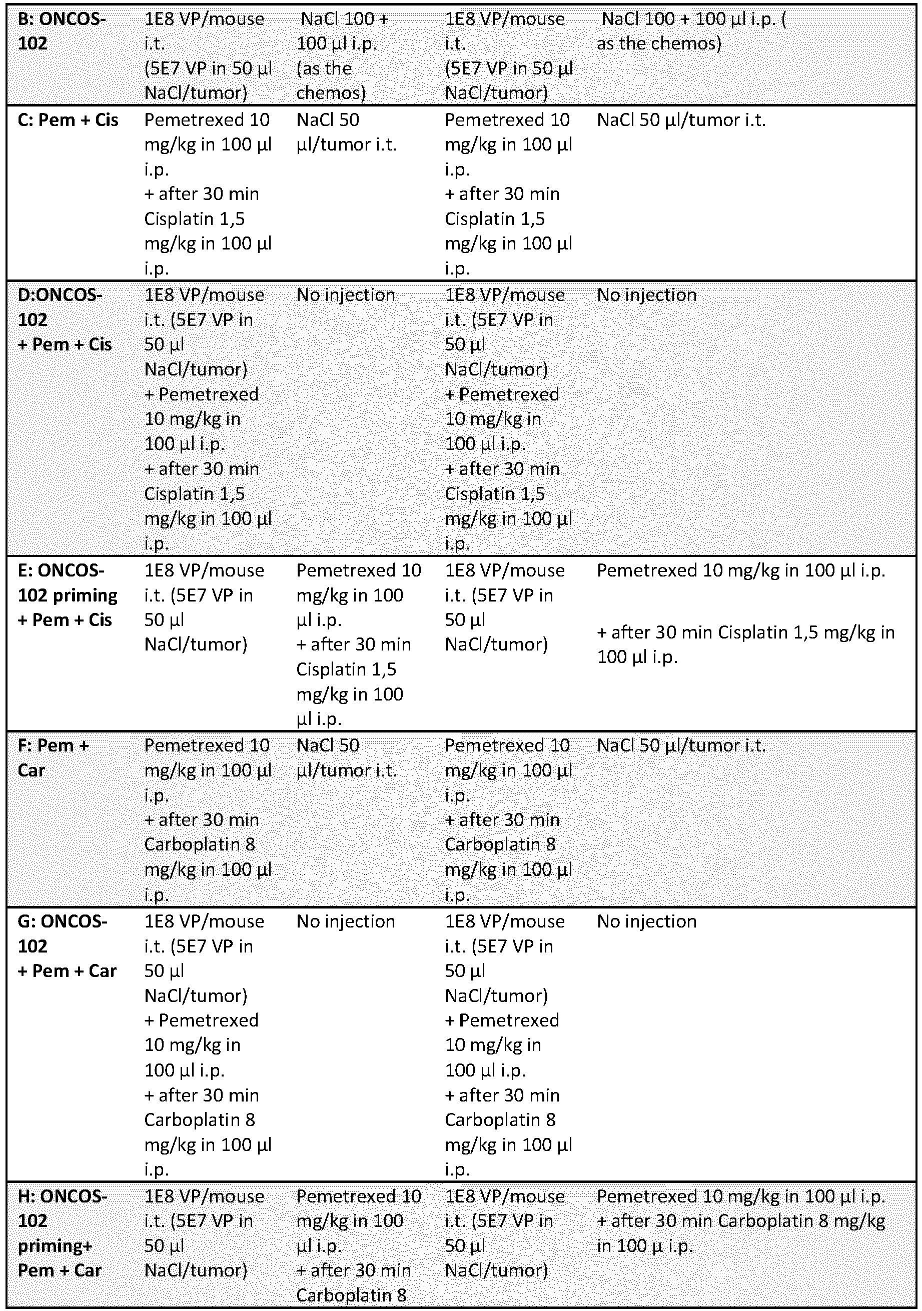

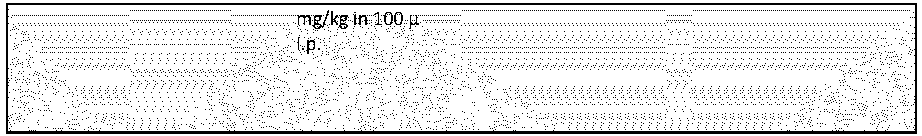

Table 2 discloses combined treatment of mesothelioma with ONCOS-102 and Pemetrexed + Cisplatin or Pemetrexed + Carboplatin in BALB/c nude mice.

**Table 2. Combined treatment of mesothelioma with ONCOS-102 and Pemetrexed + Cisplatin or Pemetrexed + Carboplatin in BALB/c nude mice. Assessment of therapeutic synergy was done with FTV calculation method. FTV (mean tumor volume (%) experimental)/(mean tumor volume control (%)). ^{∗}(Mean FTV of Chemotherapy (%)) × (mean FTV of ONCOS-102 (%)). **(expected FTV divided by the observed FTV). A ratio (R) of >1 indicates a synergistic effect, and a ratio of <1 indicates a less than additive effect.**

| **Fractional tumor volume (FTV)** | | | | | | | | |
|---|---|---|---|---|---|---|---|---|
| | | | **ONCOS-102+ Pem +Cis** | | | **ONCOS-102 rim+Pem+Cis** | | |
| Day | **Pem+Cis** | **ONCOS-102** | Exp.* | Obs.** | Ratio | Exp.* | Obs.** | Ratio |
| 21 | 1,12 | 0,60 | 0,72 | 0,40 | **1,80** | 0,72 | 0,54 | **1.32** |
| 48 | 1,28 | 0,45 | 0,57 | 0,26 | **2,19** | 0,57 | 0,23 | **2.50** |
| 60 | 1,19 | 0,44 | 0,52 | 0,29 | 1,78 | 0,52 | 0,20 | **2.5** |

| | | | **ONCOS-102+Pem+Car** | | | **ONCOS-102 rim+Pem+Car** | | |
|---|---|---|---|---|---|---|---|---|
| Day | **Pem+Car** | **ONCOS-102** | Exp.* | Obs.** | Ratio | Exp.* | Obs.** | Ratio |
| 21 | 1,32 | 0,60 | 0,79 | 0,79 | 1,0 | 0,79 | 0,85 | 0,93 |
| 48 | 1,44 | 0,45 | 0,64 | 0,38 | **1,70** | 0,64 | 0,46 | **1,41** |
| 60 | 1,42 | 0,44 | 0,62 | 0,35 | **1,8** | 0,62 | 0,32 | **1.94** |

### EXAMPLE 5. ONCOS-102 replicates locally in tumor tissues and produces human GM-CSF

Quantification for adenovirus E4 copy and the level of GM-CSF in murine organs (tumor, liver) and serum was done by qPCR and ELISA. ONCOS-102 was present only in tumors (locally), while no viral particles were detected in serum or liver in all tested groups (Fig. 4D). ONCOS-102 was producing GM-CSF protein, which was detected in the highest concentration in tumor in 5 tested groups (see Fig. 4C).

### REFERENCES

Fueyo J., Gomez-Manzano C., Alemany R., Lee P., McDonnell TJ., Mitlianga P. Shi Y-X., Levin VA., Yung WKA. and Kyritsis AP. A mutant oncolytic adenovirus targeting the Rb pathway produces anti-glioma effect in vivo. Oncogene (2000) 19:2-12.
Heise C., Hermiston T., Johnson L., Brooks G., Sampson-Johannes A., Williams A., Hawkins L. & Kirn D. An adenovirus E1A mutant that demonstrates potent and selective systemic anti-tumoral efficacy. Nat Med (2000) 6:1134-9.
Hanahan D, Weinberg RA. (2000). The hallmarks of cancer. Cell (2000) 100: 57-70.
Koski A, Kangasniemi L, Escutenaire S, Pesonen S, Cerullo V, Diaconu I, Nokisalmi P, Raki M, Rajecki M, Guse K, Ranki T, Oksanen M, et al. Treatment of cancer patients with a serotype 5/3 chimeric oncolytic adenovirus expressing GMCSF. Mol Ther (2010) 18: 1874-84.
Ranki T, Joensuu T, Jäger E, Karbach J, Wahle C, Kairemo K, Alanko T, Partanen K, Turkki R, Linder N, Lundin J, Ristimäki A, et al. Local treatment of a pleural mesothelioma tumor with ONCOS-102 induces a systemic antitumor CD8+T-cell response, prominent infiltration of CD8+lymphocytes and Th1 type polarization. OncoImmunology (2014) 3: e958937.
Siurala M, Bramante S, Vassilev L, Hirvinen M, Parviainen S, Tahtinen S, Guse K, Cerullo V, Kanerva A, Kipar A, Vaha-Koskela M, Hemminki A. Oncolytic adenovirus and doxorubicin-based chemotherapy results in synergistic antitumor activity against soft-tissue sarcoma. Int J Cancer (2015) 136: 945-54.

## Claims

1. ONCOS-102 adenovirus (Ad5/3-d24-GMCSF) for use in the treatment of human malignant mesothelioma, wherein the virus is administered in combination with two chemotherapeutic agents to a patient, wherein the two chemotherapeutic agents are either Pemetrexed and Cisplatin, or Pemetrexed and Carboplatin.

2. ONCOS-102 adenovirus for use in the treatment of human malignant mesothelioma according to claim 1, wherein the molar ratio between Pemetrexed and Cisplatin is from 10:0.75 to 10:3.

3. ONCOS-102 adenovirus for use in the treatment of human malignant mesothelioma according to claim 2, wherein the molar ratio between Pemetrexed and Cisplatin is 10:1.5.

4. ONCOS-102 adenovirus for use in the treatment of human malignant mesothelioma according to claim 1, wherein the molar ratio between Pemetrexed and Carboplatin is from 5:8 to 5:2.

5. ONCOS-102 adenovirus for use in the treatment of human malignant mesothelioma according to claim 4, wherein the molar ratio between Pemetrexed and Carboplatin is 5:4.

6. ONCOS-102 adenovirus for use in the treatment of human malignant mesothelioma according to any of claims 1 to 5, wherein the amount of the virus is from 5 × 10¹⁰ to 5 × 10¹¹ VP.

7. ONCOS-102 adenovirus for use in the treatment of human malignant mesothelioma according to claim 6, wherein the virus is administered in an amount of 3 × 10¹¹ VP/5 ml.

8. ONCOS-102 adenovirus for use in the treatment of human malignant mesothelioma according to any of claims 1 to 7, wherein the chemotherapeutic agents and the virus are administered in effective amounts.

9. ONCOS-102 adenovirus for use in the treatment of human malignant mesothelioma according to any of claims 1 to 8, wherein the virus is administered intraperitoneally, intrapleurally or by direct injection into tumor and the two chemotherapeutic agents are administered intravenously or intraperitoneally.

10. ONCOS-102 adenovirus for use in the treatment of human malignant mesothelioma according to any of claims 1 to 9, wherein the virus is administered before administering of the two chemotherapeutic agents and also during the administering period of said chemotherapeutic agents.

11. ONCOS-102 adenovirus for use in the treatment of human malignant mesothelioma according to any of claims 1 to 9, wherein the two chemotherapeutic agents are administered before the virus is administered and also during the administering period of said virus.

12. ONCOS-102 adenovirus for use in the treatment of human malignant mesothelioma according to any of claims 1 to 11, further comprising administration of one, two, three or four and any combination thereof from the list of agents consisting of Cyclohexamide, Folic acid, Cyanocobolamin and Dexamethasone to the patient.

13. ONCOS-102 adenovirus for use in the treatment of human malignant mesothelioma according to any of claims 1 to 11, further comprising administration of cyclophosphamide.

14. ONCOS-102 adenovirus for use in the treatment of human malignant mesothelioma according to any of claims 1 to 11, further comprising administration of check-point inhibitors, such as anti-PD-1/PD-L1 or anti-CTLA4 antibodies.

## Patentansprüche

1. Adenovirus ONCOS-102 (Ad5/3-d24-GMCSF) zur Verwendung in der Behandlung eines humanen malignen Mesothelioms, wobei das Virus einem Patienten in Kombination mit zwei chemotherapeutischen Mitteln verabreicht wird, wobei die zwei chemotherapeutischen Mittel entweder Pemetrexed und Cisplatin oder Pemetrexed und Carboplatin sind.

2. Adenovirus ONCOS-102 zur Verwendung in der Behandlung eines humanen malignen Mesothelioms nach Anspruch 1, wobei das Molverhältnis zwischen Pemetrexed und Cisplatin von 10:0,75 bis 10:3 beträgt.

3. Adenovirus ONCOS-102 zur Verwendung in der Behandlung eines humanen malignen Mesothelioms nach Anspruch 2, wobei das Molverhältnis zwischen Pemetrexed und Cisplatin 10:1,5 beträgt.

4. Adenovirus ONCOS-102 zur Verwendung in der Behandlung eines humanen malignen Mesothelioms nach Anspruch 1, wobei das Molverhältnis zwischen Pemetrexed und Carboplatin von 5:8 bis 5:2 beträgt.

5. Adenovirus ONCOS-102 zur Verwendung in der Behandlung eines humanen malignen Mesothelioms nach Anspruch 4, wobei das Molverhältnis zwischen Pemetrexed und Carboplatin 5:4 beträgt.

6. Adenovirus ONCOS-102 zur Verwendung in der Behandlung eines humanen malignen Mesothelioms nach einem der Ansprüche 1 bis 5, wobei die Menge des Virus von 5 × 10¹⁰ bis 5 × 10¹¹ VP beträgt.

7. Adenovirus ONCOS-102 zur Verwendung in der Behandlung eines humanen malignen Mesothelioms nach Anspruch 6, wobei das Virus in einer Menge von 3 × 10¹¹ VP/5 ml verabreicht wird.

8. Adenovirus ONCOS-102 zur Verwendung in der Behandlung eines humanen malignen Mesothelioms nach einem der Ansprüche 1 bis 7, wobei die chemotherapeutischen Mittel und das Virus in wirksamen Mengen verabreicht werden.

9. Adenovirus ONCOS-102 zur Verwendung in der Behandlung eines humanen malignen Mesothelioms nach einem der Ansprüche 1 bis 8, wobei das Virus intraperitoneal, intrapleural oder durch Direktinjektion in den Tumor verabreicht wird und die beiden chemotherapeutischen Mittel intravenös oder intraperitoneal verabreicht werden.

10. Adenovirus ONCOS-102 zur Verwendung in der Behandlung eines humanen malignen Mesothelioms nach einem der Ansprüche 1 bis 9, wobei das Virus vor der Verabreichung der beiden chemotherapeutischen Mittel und auch während des Verabreichungszeitraums der chemotherapeutischen Mittel verabreicht wird.

11. Adenovirus ONCOS-102 zur Verwendung in der Behandlung eines humanen malignen Mesothelioms nach einem der Ansprüche 1 bis 9, wobei die beiden chemotherapeutischen Mittel vor der Verabreichung des Virus und auch während des Verabreichungszeitraums des Virus verabreicht werden.

12. Adenovirus ONCOS-102 zur Verwendung in der Behandlung eines humanen malignen Mesothelioms nach einem der Ansprüche 1 bis 11, ferner umfassend die Verabreichung von einem, zwei, drei oder vier und eine beliebige Kombination von Mitteln aus der Liste von Mitteln bestehend aus Cyclohexamid, Folsäure, Cyanocobalamin und Dexamethason an den Patienten.

13. Adenovirus ONCOS-102 zur Verwendung in der Behandlung eines humanen malignen Mesothelioms nach einem der Ansprüche 1 bis 11, ferner umfassend die Verabreichung von Cyclophosphamid.

14. Adenovirus ONCOS-102 zur Verwendung in der Behandlung eines humanen malignen Mesothelioms nach einem der Ansprüche 1 bis 11, ferner umfassend die Verabreichung von Checkpoint-Inhibitoren, wie anti-PD-1/PD-L1- oder anti-CTLA4-Antikörpern.

## Revendications

1. Adénovirus ONCOS-102 (Ad5/3-d24-GMCSF) pour l'utilisation dans le traitement d'un mésothéliome maligne humain, ledit virus étant administré à un sujet en combinaison avec deux agents chimothérapeutiques, lesdits deux agents chimothérapeutiques étant ou le pémétrexed et le cisplatine ou le pémétrexed et le carboplatine.

2. Adénovirus ONCOS-102 pour l'utilisation dans le traitement d'un mésothéliome maligne humain selon la revendication 1, dans lequel le rapport molaire entre le pémétrexed et le cisplatine est de 10:0,75 à 10:3.

3. Adénovirus ONCOS-102 pour l'utilisation dans le traitement d'un mésothéliome maligne humain selon la revendication 2, dans lequel le rapport molaire entre le pémétrexed et le cisplatine est de 10:1,5.

4. Adénovirus ONCOS-102 pour l'utilisation dans le traitement d'un mésothéliome maligne humain selon la revendication 1, dans lequel le rapport molaire entre le pémétrexed et le carboplatine est de 5:8 à 5:2.

5. Adénovirus ONCOS-102 pour l'utilisation dans le traitement d'un mésothéliome maligne humain selon la revendication 4, dans lequel le rapport molaire entre le pémétrexed et le carboplatine est de 5:4.

6. Adénovirus ONCOS-102 pour l'utilisation dans le traitement d'un mésothéliome maligne humain selon l'une des revendications 1 à 5, dans lequel la quantité du virus est de 5 × 10¹⁰ à 5 × 10¹¹ pv.

7. Adénovirus ONCOS-102 pour l'utilisation dans le traitement d'un mésothéliome maligne humain selon la revendication 6, dans lequel le virus est administré dans une quantité de 3 × 10¹¹ pv/5 ml.

8. Adénovirus ONCOS-102 pour l'utilisation dans le traitement d'un mésothéliome maligne humain selon l'une des revendications 1 à 7, dans lequel les agents chimothérapeutiques et le virus sont administrés dans des quantités effectives.

9. Adénovirus ONCOS-102 pour l'utilisation dans le traitement d'un mésothéliome maligne humain selon l'une des revendications 1 à 8, dans lequel le virus est administré par voie intrapéritonéale, intrapleurale ou par injection directe dans la tumeur et les deux agents chimothérapeutiques sont administrés par voie intraveineuse ou intrapéritonéale.

10. Adénovirus ONCOS-102 pour l'utilisation dans le traitement d'un mésothéliome maligne humain selon l'une des revendications 1 à 9, dans lequel le virus est administré avant l'administration des deux agents chimothérapeutiques et également pendant la période d'administration desdits agents chimothérapeutiques.

11. Adénovirus ONCOS-102 pour l'utilisation dans le traitement d'un mésothéliome maligne humain selon l'une des revendications 1 à 9, dans lequel les deux agents chimothérapeutiques sont administrés avant l'administration du virus et également pendant la période d'administration dudit virus.

12. Adénovirus ONCOS-102 pour l'utilisation dans le traitement d'un mésothéliome maligne humain selon l'une des revendications 1 à 11, comprenant également l'administration au sujet de l'un, de deux, de trois ou de quatre et d'une combinaison quelconque d'agents de la liste d'agents constituée par le cyclohexamide, l'acide folique, le cyanocobalamine et le dexaméthasone.

13. Adénovirus ONCOS-102 pour l'utilisation dans le traitement d'un mésothéliome maligne humain selon l'une des revendications 1 à 11, comprenant également l'administration du cyclophosphamide.

14. Adénovirus ONCOS-102 pour l'utilisation dans le traitement d'un mésothéliome maligne humain selon l'une des revendications 1 à 11, comprenant également l'administration d'inhibiteurs de checkpoint tels que les anticorps anti-PD-1/PD-L1 ou anti-CTLA4.
